(19) Europäisches Patentamt
European Patent Office
Office européen des brevets

(11) Publication number: 0 228 061 A2

(12) **EUROPEAN PATENT APPLICATION**

(21) Application number: 86117844.0

(51) Int. Cl.[4]: C07D 501/36 , A61K 31/545

(22) Date of filing: 20.12.86

(30) Priority: 23.12.85 JP 291056/85
30.09.86 JP 234035/86

(43) Date of publication of application:
08.07.87 Bulletin 87/28

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Nishimura, Tatsuo
16-5, Miyamacho 4-chome
Toyonaka Osaka 560(JP)
Inventor: Yoshimura, Yoshinobu
C-601, 19 Mihogaoka
Ibaraki Osaka 567(JP)
Inventor: Hashimoto, Naoto
4-15, Tsukumodai 4-chome Suita
Osaka 565(JP)
Inventor: Miyake, Akio
30-22, Ominemotomachi 2-chome
Hirakata Osaka 573(JP)

(74) Representative: von Kreisler, Alek, Dipl.-Chem. et al
Deichmannhaus am Hauptbahnhof
D-5000 Köln 1(DE)

(54) **Cephem compounds.**

(57) A cephem compound of the general formula:

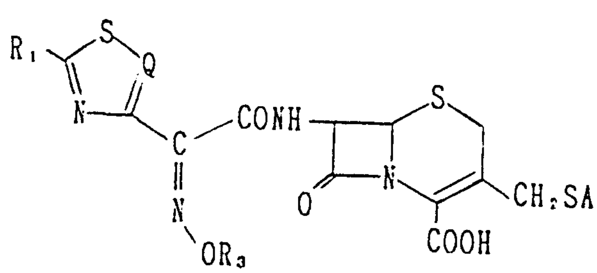

, wherein $R_1$ is an amino group which may be protected, Q is nitrogen or a group of $C-R_2$ in which $R_2$ is hydrogen or a halogen,
$R_3$ is hydrogen or a hydrocarbon residue which may be substituted,
A is a triazolopyridinyl, triazolopyridazinyl or triazolopyrazinyl group which may be substituted,
, or a salt or ester thereof

EP 0 228 061 A2

, and a process for preparing the same and a pharmaceutical composition thereof are disclosed.

## CEPHEM COMPOUNDS

This invention relates to novel cephem compounds having an excellent antimicrobial activity and a process for preparing the same and a pharmaceutical composition thereof.

Only a few cephem compounds or derivatives thereof having both of a nitrogen-containing condensed heterocyclic thiomethyl group at the 3 position and 2-(2-aminothiazol-4-yl)-or 2-(5-amino-1,3,4-thiadiazol-3-yl)-2-hydroxy (or substituted hydroxy) iminoacetamido group at the 7 position have been synthesized. Especially, the cephem compounds in which a nitrogen-containing condensed heterocyclic thiomethyl group at the 3 position is a condensed ring of triazole with another heterocyclic ring have been disclosed in Japanese Unexamined Patent Publication No. Sho60(1985)-142987 (corresponding EP-150507 A) only.

Cephem antibiotics are widely used for the treatment of diseases caused by pathogenic bacteria in man and animals; for example they are particularly useful for treatment of diseases caused by bacteria resistant to penicillin antibiotics and for treatment in penicillin-sensitive patients. In these cases cephem antibiotics effective both against Gram-positive bacteria and against Gram-negative bacteria are desirable, and therefore cephem antibiotics having broad antibacterial spectra have been studied actively. Cephem antibiotics which are active against resistant bacteria which had been experienced with penicillin, i.e., so-called cephalosporin-resistant bacteria, have been desired, and cephem antibiotics having an antibacterial activity high enough for clinical use against some Escherichia coli resistant to the known cephalosporins, some of the genus Citrobacter, most of the genus Proteus which is indole-positive, and pathogenic bacteria classified into the genus Enterobacter, the genus Serratia and the genus Pseudomonas have also been investigated actively. Such studies have suggested that cephem compounds having a nitrogen-containing heterocyclic thiomethyl group at the 3 position together with a nitrogen-containing heterocyclic amino group or an acylamino group at the 7 position, or oxa derivatives or carba derivatives thereof, have more excellent antimicrobial activity with unique antibacterial spectra, and various such compounds have been synthesized (e.g., see USP 3,907,786; USP 3,946,000; USP 4,317,907; USP 4,385,178; USP 4,358,448 and USP 4,463,003).

This invention relates to a cephem compound of the general formula:

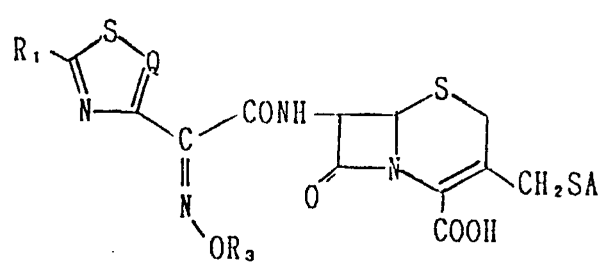

(I)

, wherein $R_1$ is an amino group which may be protected,

Q is nitrogen or a group of $C\text{-}R_2$ in which $R_2$ is hydrogen or a halogen,

$R_3$ is hydrogen or a hydrocarbon residue which may be substituted,

A is a triazolopyridinyl, triazolopyridazinyl or triazolopyrazinyl group which may be substituted,

, or a pharmaceutically acceptable salt or ester thereof, and to a process for preparing the same and a pharmaceutical composition thereof.

As described above, only a few cephem compounds or derivatives thereof having both of a nitrogen-containing condensed heterocyclic thiomethyl group at the 3 position and aminothiazolyl (or aminothiadiazolyl) oxyiminoacetamido group at the 7 position have been synthesized. Especially, compounds of this invention having triazolopyridinylthiomethyl, triazolopyridazinylthiomethyl or triazolopyradinylthiomethyl at the 3 position and 2-(2-aminothiazol-4-yl)-, 2-(2-amino-5-halogenothiazol-4-yl)-or 2-(5-amino-1,2,4-thiadiazol-3-yl)-2-hydroxy (or substituted hydroxy) iminoacetamido at the 7 position have never been synthesized. The inventors have succeeded in synthesizing the compound of the general formula (I) having these chemical structural characteristics, and found from studies on the antibacterial activity and spectra, that the compound (I) has an excellent antibacterial activity against a variety of bacteria, thus completing this invention. Some of the compounds (I) have also strong antibacterial activity against cephalosporin resistant beacteria described above.

The substituent $R_1$ in the above mentioned cephem compound (I) represents an amino group which may be protected, i.e., amino group or a protected amino group. In the field of synthesis of $\beta$-lactam and peptide, protecting groups for amino group have been extensively studied, and the method of protection has already been established. In the present invention as well, any of those known amino-protecting groups may suitably be employed. As such amino-protecting groups, there may be mentioned for example $C_{6-10}$ aryl-acyl group, $C_{1-5}$ alkanoyl group, $C_{1-5}$ alkenoyl group, $C_{6-10}$ arylsulfonyl group, $C_{1-10}$ alkylsulfonyl group, substituted oxycarbonyl group, carbamoyl group, thiocarbamoyl group, $C_{6-10}$ aryl-methyl group, $C_{6-10}$ aryl-methylene group, $C_{6-10}$ arylthio group, substituted silyl group or 2-$C_{1-8}$ alkoxy-carbonyl-1-methyl-2-ethenyl group.

As the $C_{6-10}$ aryl-acyl group, there may be specifically mentioned benzoyl, naphtoyl or phthaloyl, the aromatic ring of which may be substituted by $C_{1-4}$ alkyl, $C_{1-6}$ alkoxy, halogen, nitro or the like.

As the substituted aryl-acyl group, there may be specifically mentioned p-toluoyl, p-tert-butylbenzoyl, p-methoxy benzoyl, p-tert-butoxybenzoyl, p-chlorobenzoyl or p-nitrobenzoyl.

As the $c_{1-5}$ alkanoyl group, there may be specifically mentioned here formyl, acetyl, propionyl, butyryl, valeryl, pivaloyl, succinyl or glutaryl, which may further be substituted by $C_{1-6}$ alkoxy, halogen, $C_{6-10}$ aryl, $C_{6-10}$ aryloxy or $C_{6-10}$ arylthio. As the substituted alkanoyl group, there may be specifically mentioned monochloroacetyl, dichloroacetyl, trichloroacetyl, monobromoacetyl, monofluoroacetyl, difluoroacetyl, trifluoroacetyl, monoiodoacetyl, 3-oxobutyryl, 4-chloro-3-oxobutyryl, phenylacetyl, p-chlorophenylacetyl, phenoxyacetyl or p-chlorophenoxyacetyl.

As the $C_{3-5}$ alkenoyl group, there may be specifically mentioned here acryloyl, crotonoyl or maleoyl, which may further be substituted by $C_{6-10}$ aryl.

An example of the substituted alkenoyl group includes cinnamoyl.

As the $C_{6-10}$ arylsulfonyl group, there may be specifically mentioned here benzenesulfonyl or naphthalenesulfonyl, the aromatic ring of which may further be substituted by $C_{1-4}$alkyl, $C_{1-6}$ alkoxy, halogen or nitro. Examples of the substituted arylsulfonyl group include p-toluenesulfonyl, p-tert-butylbenzenesulfonyl, p-methoxybenzenesulfonyl, p-chlorobenzenesulfonyl or p-nitrobenzenesulfonyl.

As the $C_{1-6}$ alkylsulfonyl group, there may be specifically mentioned methanesulfonyl, ethanesulfonyl or camphorsulfonyl, which may further be substituted by $C_{6-10}$ aryl, $C_{6-10}$ aryloxy or halogen.

As the substituted oxycarbonyl group, there may be specifically mentioned $C_{2-8}$ alkoxy-carbonyl group, such as methoxycarbonyl, ethoxycarbonyl, isopropoxycarbonyl, tertbutoxycarbonyl or isobornyloxycarbonyl, or $C_{7-12}$ aralkyloxycarbonyl, such as benyloxycarbonyl. The $C_{1-8}$ alkoxy-carbonyl group may be further substituted by $C_{1-6}$ alkoxy, $C_{2-5}$ alkanoyl, substituted silyl, $C_{1-4}$ alkylsulfonyl, halogen or cyano.

Examples of the substituted alkoxy-carbonyl group include methoxymethyloxycarbonyl, acetylmethyloxycarbonyl, 2-trimethylsilylethoxycarbonyl, 2-methanesulfonylethoxycarbonyl, 2,2,2-trichloroethoxycarbonyl or 2-cyanoethoxycarbonyl.

The aromatic ring in the $C_{6-10}$ aryloxycarbonyl group and the $C_{7-10}$ aralkyloxycarbonyl group may be substituted by $C_{1-4}$ alkyl, $C_{1-6}$ alkoxy, halogen or nitro. Examples of the substituted aryloxycarbonyl group include p-methylphenoxycarbonyl, p-methoxyphenoxycarbonyl and p-chlorophenoxycarbonyl. Examples of the substituted aralkyloxycarbonyl group include p-methylbenzyloxycarbonyl, p-methoxybenzyloxycarbonyl, p-chlorobenzyloxycarbonyl and p-nitrobenzyloxycarbonyl.

Also, the alkyl group of the $C_{7-10}$ aralkyloxycarbonyl group may be substituted by $C_{6-10}$aryl or halogen. Its example is benzhydryloxycarbonyl.

Carbamoyl group may be substituted. Examples of the substituted carbamoyl group include N-methylcarbamoyl, N-ethylcarbamoyl, N,N-dimethylcarbamoyl, N-phenylcarbamoyl or N-(p-methoxyphenyl)-carbamoyl.

Similarly, thiocarbamoyl group may be substituted. An example of the substituted thiocarbamoyl group includes N-methylthiocarbamoyl.

As the $C_{6-10}$ aryl-methyl group, there may be specifically mentioned benzyl or naphthyl-methyl, the aromatic ring of which may be substituted by $C_{1-4}$ alkoxy, halogen or nitro. Examples of the substituted aryl-methyl group include p-methylbenzyl, p-methoxybenzyl, p-chlorobenzyl or p-nitrobenzyl. The methyl group of the aryl-methyl group may be further substituted by other one or two $C_{6-10}$ aryl groups, to form for example benzhydryl or trityl.

As the $C_{6-10}$aryl-methylene group, there may be specifically mentioned benzylidene, the aromatic ring of which may be substituted by $C_{1-4}$ alkyl, $C_{1-6}$ alkoxy, halogen or nitro. Examples of the substituted aryl-methylene groups are p-methylbenhylidene or p-chlorobenzylidene.

As the $C_{6-10}$ arylthio group, there may be specifically mentioned o-nitro-phenylthio.

The substituted silyl group means a silyl group, together with the amino group to be protected, representable by the general formula; $R_6R_7R_8SiNH$, $(R_6R_7R_8Si)_2N$ or

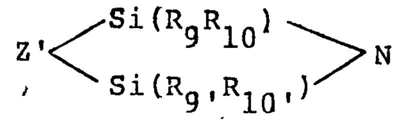

[wherein $R_6$, $R_7$, $R_8$, $R_9$, $R_{10}$, $R_9'$ and $R_{10}'$may be the same or different and stand for $C_{1-4}$ alkyl group, such as methyl, ethyl or tert-butyl, or $C_{6-10}$ aryl group, such as phenyl, and Z′ stands for $C_{1-3}$ alkylene group e.g., methylene, ethylene or propylene], which is exemplified by trimethylsilyl, tert-butyldimethylsilyl or $-Si(CH_3)_2CH_2CH_2Si(CH_3)_2-$.

The $C_{1-8}$ alkoxy group of the 2-$C_{1-8}$alkoxycarbonyl-1-methyl-1-ethenyl group may be exemplified by methoxy, ethoxy or tert-butoxy.

In the above mentioned cephem compound (I), the symbol Q is nitrogen atom or a group C-$R_2$ where $R_2$ is hydrogen or a halogen atom. As halogen atom there may be mentioned fluorine, chlorine, bromine or iodine, among which chlorine is preferred.

In the above cephem compound (I), the substituent $R_3$ is hydrogen atom or a hydrocarbon residue which may be subsituted. The hydrocarbon residue may be exemplified by $C_{1-6}$alkyl group, $C_{2-6}$ alkenyl group, $C_{3-6}$ cycloalkyl group, especially preferably, $C_{1-3}$ alkyl group, (e.g., methyl, ethyl or propyl) or a substituted C $_{1-3}$ alkyl group. The $C_{1-6}$ alkyl group may be exemplified by methyl, ethyl, n-propyl, isopropyl, n-butyl, isobutyl, sec-butyl, tert-butyl, pentyl or hexyl. The $C_{2-6}$ alkenyl group may be exemplified by vinyl, allyl, isopropenyl, methallyl, 1,1-dimethylallyl, 2-butenyl or 3-butenyl. The $C_{3-6}$ cycloalkyl group may be exemplified by cyclopropyl, cyclobutyl, cyclopentyl or cyclohexyl.

Substituents of these hydrocarbon residues may be exemplified by hydroxyl group, $C_{3-6}$ cycloalkyl group, $C_{1-6}$alkoxy group, $C_{3-6}$ cycloalkloxy group, $C_{6-10}$ aryloxy group, $C_{7-12}$aralkyloxy group, mercapto group, $C_{1-6}$alkylthio group, $C_{3-6}$cycloalkylthio group, $C_{6-10}$ arylthio group, $C_{7-12}$ aralkylthio group, amino group, mono-C $_{1-4}$ alkylamino group, di-$C_{1-4}$ alkylamino group, $C_{3-6}$ cycloalkylamino group, $C_{6-10}$ arylamino group, $C_{7-12}$ aralkylamino group, cyclic amino group, azido group, nitro group, halogen atom, cyano group, carboxyl group, $C_{1-8}$ alkoxycarbonyl group, $C_{6-10}$ aryloxy-carbonyl group, C $_{3-6}$ cycloalkyloxy-carbonyl group, $C_{7-12}$ aralkyloxy-carbonyl group, $C_{6-10}$ aryl-acyl group, $C_{1-5}$ alkanoyl group, $C_{3-5}$ alkenoyl group, $C_{6-10}$ aryl-acyloxy group, $C_{2-5}$ alkanoyloxy group, $C_{3-5}$ alkenoyloxy group, carbamoyl group, substituted carbamoyl group, thiocarbamoyl group, substituted thiocarbamoyl group, carbamoyloxy group, substituted carbamoyloxy group, phthalimido group, $C_{2-5}$ alkanoylamino group, $C_{6-10}$ aryl-acyl amido group, $C_{1-4}$ alkoxycarbonylamino group, $C_{6-10}$ aryloxy-carbonylamino group, $C_{7-12}$ aralkyloxy-carbonylamino group or heterocyclic group. More specifically, the $C_{3-6}$ cycloalkyl group is cyclopropyl, cyclobutyl, cyclopentyl or cyclohexyl; the $C_{1-6}$ alkoxy group is methoxy, ethoxy, n-propoxy, iso-propoxy, n-butoxy or tert-butoxy; the $C_{3-6}$ cycloalkyloxy group is cyclopropyloxy or cyclohexyloxy; the $C_{6-10}$ aryloxy group is phenoxy or naphthyloxy; the $C_{7-12}$ aralkyloxy group is benzyloxy or 2-phenylethyloxy; the $C_{1-6}$ alkylthio group is methylthio, ethylthio, n-propylthio or n-butylthio; the $C_{3-6}$ cycloalkylthio group is cyclopropylthio or cyclohexylthio; the $C_{6-10}$ arylthio group is phenylthio; the $C_{7-12}$aralkylthio group is benzylthio; the mono-$C_{1-4}$ alkylamino group is methylamino, ethylamino, n-propylamino or n-butylamino; the di-$C_{1-4}$ alkylamino group is dimethylamino, diethylamino, methylethylamino, di-(n-propyl)amino or di(n-butyl)amino; the $C_{3-6}$ cycloalkylamino group is cyclopropylamino or cyclohexylamino; the $C_{6-10}$ arylamino group is anilino; the $C_{7-12}$ aralkylamino group is benzylamino or 2-phenylethylamino; the cyclic amino group is pyrrolidino, piperidino, piperazino, morpholino or 1-pyrrolyl, 1-imidazolyl or 1-pyrazolyl; the halogen atom is fluorine, chlorine or bromine; the $C_{1-8}$ alkoxy-carbonyl group is methoxycarbonyl, ethoxycarbonyl, n-propoxycarbonyl, isopropoxycarbonyl, n-butoxycarbonyl, isobutoxycarbonyl, tert-butoxycarbonyl or isobornyloxycarbonyl; the $C_{6-10}$ aryloxy-carbonyl group is phenoxycarbonyl; the $C_{3-6}$ cycloalkyloxy-carbonyl group is cyclopropyloxycarbonyl or cyclohexyloxycarbonyl; the $C_{7-12}$aralkyloxy-carbonyl group is benzyloxycarbonyl; the $C_{6-10}$aryl-acyl group is benzoyl, phthaloyl or phenylacetyl; the $C_{1-6}$ alkanoyl group is formyl, acetyl, propionyl, butyryl, valeryl or pivaloyl; the $C_{3-5}$ alkenoyl group is acryloyl, crotonoyl or maleoyl; the $C_{6-10}$ aryl-acyloxy group is benzoyloxy; the $C_{2-5}$ alkanoyloxy group is acetoxy, propionyloxy, butyryloxy, valeryloxy or pivaloyloxy; the $C_{3-5}$ alkenoyloxy group is acryloyloxy; the substituted carbamoyl group is N-methylcarbamoyl, N,N-dimethylcarbamoyl, N-ethylcarbamoyl, N-phenylcarbamoyl, pyrrolidinocarbonyl, piperidinocarbonyl, piperazinocarbonyl or morpholinocarbonyl; the substituted thiocarbamoyl group is N-methylthiocarbamoyl; the substituted carbamoyloxy group is N-methylcarbamoyloxy, N,N-dimethylcarbamoyloxy or N-ethylcar-

bamoyloxy; the C $_{2\text{-}5}$ alkanoylamido group is acetamido or propionamido; the C $_{6\text{-}10}$ aryl-acyl amino group is benzamido; the $C_{1\text{-}4}$ alkoxy-carbonylamino group is methoxycaronylamino, ethoxycarbonylamino or tert-butoxycarbonylamino; the $C_{6\text{-}10}$ aryloxy-carbonylamino group is phenoxycarbonylamino; the $C_{7\text{-}12}$ aralkyloxy-carbonylamino group is benzyloxycarbonylamino; and the heterocyclic group means a 5-or 6-membered ring containing one to several hetero atoms, such as nitrogen atom (which may be oxidized), oxygen atom or sulfur atom, or a condensed ring thereof, which possesses a binding arm at carbon atom; such as 2-pyridyl, 3-pyridyl, 4-pyridyl, N-oxido-2-pyridyl, N-oxido-3-pyridyl, N-oxido-4-pyridyl, 2-piperidinyl, 3-piperidinyl, 4-piperidinyl, 2-pyrazinyl, 2-pyrimidinyl, 4-pyrimidinyl, 5-pyrimidinyl, 3-pyridazinyl, 4-pyridazinyl, 2-pyranyl, 3-pyranyl, 4-pyranyl, 2-thiopyranyl, 3-thiopyranyl, 4-thiopyranyl, 3H-indol-2-yl, 3H-indol-3-yl, 1,2,3-thiadiazol-4-yl, 1,2,4-thiadiazol-3-yl, 1,3,4-thiadiazol-2 or 5-yl, 1,2,5-thiadiazol-3 or 4-yl, 1,2,3-triazol-4 or 5-yl 1,2,4-triazol-3 or 5-yl, 1H-tetrazol-5-yl, 2-benzopyranyl, 2-furyl, 3-furyl, 2-thienyl, 3-thienyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isoxazolyl, 3-isothiazolyl, 4-isothiazolyl, 5-isothiazolyl, 2-pyrrolyl, 3-pyrrolyl, 2-pyrrolidinyl, 3-pyrrolidinyl, 2-imidazolyl, 4-imidazolyl, 5-imidazolyl, 3-pyrazolyl, 4-pyrazolyl or 5-pyrazolyl.

The alkyl group constituting the aralkyl group in the above mentioned aralkyloxy, aralkylthio, aralkylamino, aralkyloxycarbonyl and aralkyloxycarbonylamino groups may be substited by another $C_{6\text{-}10}$ aryl group, to form specifically benzhydryloxy, benzhydrylthio, benzhydryloxycarbonyl and benzhydryloxycarbonylamino.

The number of the substituents on the above mentioned hydrocarbon residue is not restricted to one and may be plural (two to four) which are the same or different. Two substituents on the $\alpha$ position of $C_{1\text{-}6}$alkyl group may form a heterocyclic ring. More preferred examples of the substituted hydrocarbon residues include $C_{1\text{-}3}$ alkyl groups substituted by halogen, hydroxy, amino, $C_{1\text{-}6}$ alkoxy, carboxyl, $C_{1\text{-}6}$ alkoxycarbonyl or cyano. Examples of the substituted hydrocarbon residues include methoxymethyl, 1-methoxyethyl, 1-ethoxyethyl, 2-hydroxyethyl, 2-aminoethyl, 2-fluoroethyl, 2-chloroethyl, 2-bromoethyl, carboxymethyl, cyanomethyl, 1-carboxy-1-methylethyl, 1-carboxycyclopropyl, methoxycarbonylmethyl, ethoxycarbonylmethyl, tert-butoxycarbonylmethyl, 1-methoxycarbonyl-1-methylethyl, 1-ethoxycarbonyl-1-methylethyl, 1-tert-butoxycarbonyl-1-methylethyl, 1-benzyloxycarbonyl-1-methylethyl, 1-pivaloyloxycarbonyl-1-methylethyl, 2-(pyrazolyl)ethyl, 2-(imidazolyl)ethyl, 2-(2-oxopyrrolidin-3-yl)ethyl, 2-amino-4(or 5)-thiazolylmethyl, 5-amino-1,2,4-thiadiadiazol-3-ylmethyl, 1-carboxy-1-(2,3,4-trihydroxyphenyl)methyl, 2-oxo-3-pyrrolidyl, and many others. In this specification, $OR_3$ group in all cases is syn configuration (Z -configuration).

The substituent A in the above mentioned cephem compound (I) represents a triazolopyridinyl, triazolopyridazinyl or triazolopyrazinyl group which may be substituted. The triazolopyridinyl, triazolopyridainyl or triazolopyrazinyl group means the group which is formed from the condensed ring of triazole ring with pyridine, pyridazine or pyrazine ring sharing a C-N bond by removing one hydrogen atom attached to any of its ring constituting carbon atoms.

Examples of triazolopyridine rings constituting the triazolopyridinyl group include

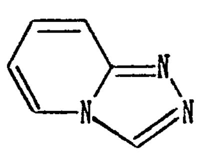 , 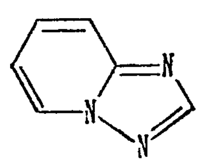 , 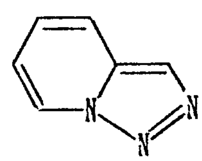

Examples of triazolopyridazine rings constituting the triazolopyridazinyl group include

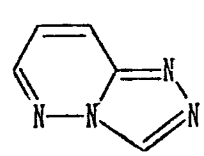 , 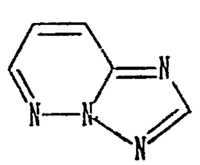 , 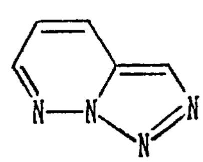

Examples of triazolopyrazine rings constituting the triazolopyrazinyl group include

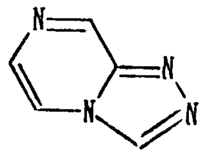 , 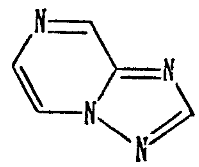 , 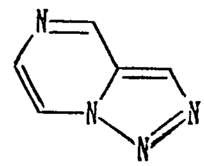

Preferred examples of the triazolopyridinyl or triazolopyridazinyl group inlcude

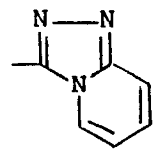 , 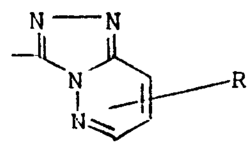

(wherein R is morpholino, phenyl, $C_{1-6}$ alkoxy, $C_{1-6}$ alkylthio or halogen) ,

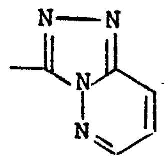 , 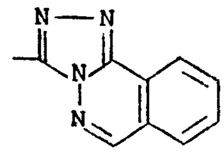 , 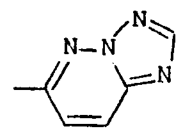 ,

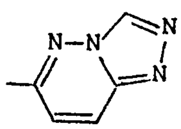 , 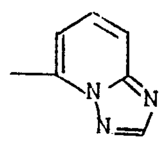 or 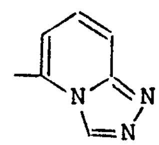

More preferred examples of the triazolopyridinyl or triazolopyridazinyl group include

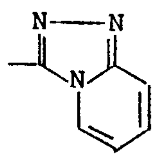 , 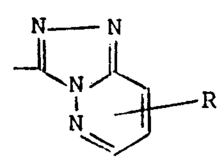

(wherein R is morpholino, phenyl, $C_{1-6}$ alkoxy, $C_{1-6}$ alkylthio or halogen) ,

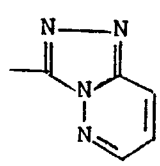 , 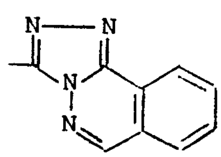 , 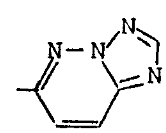 or 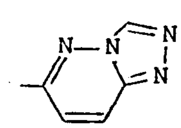

The substituent A means a group having a binding arm at any of carbon atoms in the above condensed ring. Also, any of hydrogen atoms attached to carbon atoms which are other than the carbon atom having binding arm in the above condensed ring group (A) may be substituted by other substituents. Examples of such substituents include hydroxyl group, hydroxy $C_{1-4}$ alkyl group, $C_{1-6}$ alkyl group, $C_{2-6}$alkenyl group, $C_{2-6}$ alkynyl group, $C_{4-6}$ alkadienyl group, $C_{3-6}$ cycloalkyl group, $C_{3-6}$ cycloalkenyl group, $C_{3-6}$cycloalkyl $C_{1-6}$ alkyl group, $C_{6-10}$ aryl group, $C_{7-12}$ aralkyl group, heterocyclic group, $C_{1-6}$ alkoxy group, $C_{1-6}$ alkoxy $C_{1-4}$ alkyl group, $C_{3-6}$ cycloalkyloxy group, $C_{6-10}$ aryloxy group, $C_{7-12}$ aralkyloxy group, mercapto group, mercapto $C_{1-6}$ alkyl group, sulfo group, sulfo $C_{1-4}$ alkyl group, $C_{1-6}$ alkylthio group, $C_{1-6}$ alkylthio $C_{1-4}$ alkyl group, $C_{3-6}$

cycloalkylthio group, $C_{6-10}$ arylthio group, $C_{7-12}$aralkylthio group, cyano $C_{2-4}$ alkylthio group, amino group, amino $C_{1-4}$ alkyl group, mono-$C_{1-4}$ alkylamino group, di-$C_{1-4}$alkylamino group, mono-$C_{1-4}$ alkylamino $C_{1-4}$ alkyl group, di-$C_{1-4}$ alkylamino $C_{1-4}$ alkyl group, C $_{3-6}$ cycloalkylamino group, $C_{6-10}$ arylamino group, $C_{7-12}$ aralkylamino group, cyclic amino group, cyclic amino C $_{1-4}$ alkyl group, cyclic amino $C_{1-4}$ alkylamino group, azido group, nitro group, halogen atom, halogeno $C_{1-4}$ alkyl group, cyano group, cyano $C_{1-4}$ alkyl group, carboxyl group, carboxy $C_{1-4}$ alkyl group, $C_{1-8}$ alkoxy-carbonyl group, $C_{1-8}$ alkoxy-carbonyl $C_{1-4}$ alkyl group, $C_{6-10}$ aryloxy-carbonyl group, $C_{3-6}$ cycloalkyloxycarbonyl group, $C_{7-12}$ aralkyloxy-carbonyl group, C $_{6-10}$ aryl-acyl group, $C_{1-5}$ alkanoyl group, C $_{2-5}$ alkanoyl $C_{1-4}$ alkyl group, $C_{3-5}$alkenoyl group, $C_{6-10}$ aryl-acyloxy group, $C_{2-5}$alkanoyloxy group, $C_{2-5}$ alkanoyloxy $C_{1-4}$ alkyl group, $C_{3-5}$ alkenoyloxy group, carbamoyl group, carbamoyl $C_{2-4}$ alkyl group, substituted carbamoyl group, thiocarbamoyl group, substituted thiocarbamoyl group, carbamoyloxy group, carbamoyloxy $C_{1-4}$alkyl group, substituted carbamoyloxy group, phthalimido group, $C_{2-5}$ alkanoylamido group, $C_{6-10}$aryl-acylamido group, sulfonamido group, 2-amino-2-carboxyethyl group, $C_{1-4}$ alkoxycarboxamido group, $C_{6-10}$ aryloxy-carboxamido group or $C_{7-12}$aralkyloxy-carboxamido group.

Specifically, the $C_{1-4}$ hydroxy alkyl group may be hydroxymethyl or 2-hydroxyethyl. The $C_{1-6}$ alkyl group may be methyl, ethyl, propyl, isopropyl, butyl, isobutyl, sec-butyl, tert-butyl, pentyl or hexyl. The $C_{2-6}$ alkenyl group may be vinyl, allyl, isopropenyl, methallyl , 1.2-dimethylallyl, 1-butenyl, 2-butenyl or 3-butenyl. The $C_{2-6}$ alkynyl group may be ethynyl, 1-propynyl or propargyl. The $C_{4-6}$alkadienyl group may be 1,3-butadienyl. The $C_{3-6}$ cycloalkyl group may be cyclopropyl, cyclobutyl, cyclopentyl or cyclohexyl. The $C_{3-6}$ cycloalkenyl group may be 1-cyclopentenyl, 2-cyclopentenyl, 3-cyclopentenyl, 1-cyclohexenyl, 2-cyclohexenyl, 3-cyclohexenyl or 1,4-cyclohexadienyl. The $C_{3-6}$cycloalkyl-$C_{1-6}$ alkyl group may be cyclopentylmethyl or cyclohexylmethyl. The $C_{6-10}$ aryl group may be phenyl or naphthyl. The $C_{7-12}$ aralkyl group may be benzyl or phenethyl. The heterocyclic group includes the same as mentioned above. The $C_{1-6}$ alkoxy group may be methoxy, ethoxy, propoxy, iso propoxy, butoxy or tert-butoxy, The $C_{1-6}$ alkoxy-$C_{1-4}$ alkyl group may be methoxymethyl, ethoxymethyl or 2-methoxyethyl. The $C_{3-6}$ cycloalkyloxy group may be cyclopropyloxy or cyclohexyloxy. The $C_{6-10}$ arlyloxy group may be phenoxy or naphthyloxy. The $C_{7-12}$ aralkyloxy group may be benzyloxy, 1-phenylethyloxy or 2-phenylethyloxy. The $C_{1-4}$ mercaptoalkyl group may be mercaptomethyl or 2-mercaptoethyl. The $C_{1-4}$ sulfoalkyl group may be sulfomethyl or 2-sulfoethyl. The $C_{1-6}$ alkylthio group may be methylthio, ethylthio, propylthio, isopropylthio or buylthio. The $C_{1-6}$ alkylthio-$C_{1-4}$alkyl group may be methylthiomethyl or 2-methylthioethyl. The $C_{3-6}$ cycloalkylthio group may be cyclopropylthio or cyclohexylthio. The C $_{6-10}$ arylthio group may be phenylthio. The $C_{7-12}$ aralkylthio group may be benzylthio. The $C_{1-4}$cyanoalkylthio group may be cyanomethylthio or cyanoethylthio. The $C_{1-4}$ aminoalkyl group may be aminomethyl or 2-aminoethyl. The mono-$C_{1-4}$ alkylamino group may be methylamino, ethylamino, propylamino or butylamino. The di-$C_{1-4}$alkylamino group may be dimethylamino, diethylamino, methylethylamino, dipropylamino or dibutylamino. The mono-$C_{1-4}$alkylamino -$C_{1-4}$ alkyl group may be methylaminomethyl, ethylaminomethyl, 2-(N-methylaminoethyl) or 3-(N-methylamino)propyl. The di-$C_{1-4}$ alkylamino -$C_{1-4}$ alkyl group may be N,N-dimethylaminomethyl, N,N-diethylaminomethyl, 2-(N,N-dimethylamino)ethyl, 2-(N,N-diethylamino)ethyl or 3-(N,N-dimethylamino)propyl. The $C_{3-6}$ cycloalkylamino group may be cyclopropylamino or cyclohexylamino. The $C_{6-10}$ arylamino group may be anilino or N-methylanilino. The $C_{7-12}$aralkylamino group may be benzylamino, 1-phenylethylamino or 2-phenylethylamino. The cyclic amino group may be pyrrolidino, piperidino, piperazino, morpholino or 1-pyrrolyl. the cyclic amino $C_{1-4}$ alkyl group may be pyrrolidinomethyl, piperidinomethyl, piperazinomethyl, morpholinomethyl, 2-(morpholino)ethyl, 1-imidazolylmethyl or 1-imidazolylethyl. The cyclic amino $C_{1-4}$ alkylamino group may be pyrrolidinomethylamino, piperidinomethylamino, piperazinomethylamino or morpholinomethylamino. The halogen atom may be fluorine, chlorine or bromine. The $C_{1-4}$ haloalkyl group may be monofluoromethyl, difluoromethyl, trifluoromethyl, 1-fluoroethyl, 2-fluoroethyl, monochloromethyl, dichloromethyl, trichloromethyl, 1-chloroethyl, 2-chloroethyl, bromomethyl or iodomethyl. The cyano -$C_{1-4}$ alkyl group may be cyanomethyl or 2-cyanoethyl. The carboxy -C $_{1-4}$ alkyl group may be carboxymethyl, 1-carboxyethyl or 2-carboxyethyl. The $C_{1-8}$ alkoxy-carbonyl group may be methoxycarbonyl, ethoxycarbonyl, propoxycarbonyl, isopropoxycarbonyl, butoxycarbonyl, tert-butoxycarbonyl or isobornyloxycarbonyl. The $C_{1-8}$ alkoxy-carbonyl-$C_{1-4}$ alkyl group may be methoxycarbonylmethyl, ethoxycarbonylmethyl or tert-butoxycarbonylmethyl. The $C_{6-10}$ aryloxy-carbonyl group may be phenoxy carbonyl. The $C_{3-6}$ cycloalkyloxy-carbonyl group may be cyclopropyloxycarbonyl or cyclohexyloxycarbonyl. The $C_{7-12}$ aralkyloxy-carbonyl group may be benzyloxycarbonyl. The $C_{6-10}$ aryl-acyl group may be benzoyl, phthaloyl or phenylacetyl. The $C_{1-5}$ alkanoyl group may be formyl, acetyl, propionyl, butyryl, valeryl or pivaloyl. The $C_{2-5}$ alkanoloxy-$C_{1-4}$ alkyl group may be acetylmethyl, 1-acetylethyl or 2-acetylethyl. The $C_{3-5}$ alkenoyl group may be acryloyl, crotonoyl or maleoyl. The $C_{6-10}$ aryl-acyloxy group may be benzoyloxy. The $C_{2-5}$ alkanoyloxy group may be acetoxy, propionyloxy, butyryloxy or pivaloyloxy. The $C_{2-5}$alkanoyloxy-$C_{1-4}$ alkyl group may be acetoxymethyl, 1-acetoxyethyl or 2-acetoxyethyl. The $C_{3-5}$ alkenoyloxy group may be acryloyloxy. The carbamoyl -$C_{1-4}$ alkyl group may be carbamoylmethyl. The substituted carbamoyl group may be N-methylcarbamoyl, N,N-dimethylcarbamoyl, N-ethylcarbamoyl, N,N-diethylcarbamoyl, N-phenylcarbamoyl, piperidinocarbonyl, piperazinocarbonyl or morpholinocarbonyl. The carbamoyloxy-$C_{1-4}$ alkyl group may be carbamoyloxymethyl. The substituted thiocarbamoyl group may be N-methylthiocarbamoyl. The substituted carbamoyloxy group may be N-methylcarbamoyloxy, N,N-dimethylcarbamoyloxy or N-ethylcarbamoyloxy. The $C_{2-5}$alkanolylamido group may be acetamido or propionamido. The $C_{6-10}$ aryl-acylamido group may be benzamido. The $C_{1-4}$ alkoxy-carbonylamino group may be methyoxycarbonylamino, ethoxycarbonylamino or tert-butoxycarbonylamino. The $C_{6-10}$ aryloxy-carbonylamino group may be phenoxycarbonylamino. The $C_{7-12}$ aralkyloxy-carbonyl amino group may be benzyloxycarbonylamino.

Also, the alkyl group in the aralkyl constituting the above mentioned $C_{7-12}$ aralkyl, $C_{7-12}$ aralkyloxy, $C_{7-12}$ aralkylthio, $C_{7-12}$ aralkylamino, $C_{7-12}$ aralkyloxy-carbonyl or $C_{7-12}$ aralkyloxy-carbonylamino group may be substituted by another $C_{6-10}$ aryl group, to form specifically benzhydryl, benzhydryloxy, benzhydrylthio, benzhydrylamino, benzhydryloxy-carbonyl or benzhydryloxycarbonylamino.

There may be the same or different plural (two to four) of the above mentioned substituents, without limiting to the case of single substituent. Two adjacent substituents on pyridine ring, pyridazine ring or pyrazine ring may combine to form an alicylic ring, aromatic ring or heterocyclic ring.

The cephem compound (I) may be a salt or ester thereof. Among the salts of the compound (I), especially pharmaceutically acceptable salts are used when the compound (I) is applied as an antimicrobial agent, and other salts are utilized as intermediates for synthesis.

Examples of the salts of the compound (I) include the inorganic base salts, ammonium salts, organic base salts, inorganic acid addition salts, organic acid addition salts, and basic amino acid salts. Inorganic bases that can form the inorganic base salts include alkaline metals (e.g., sodium, potassium), and alkaline earth metals (e.g, calcium); organic bases that can form the organic base salts include procaine, 2-phenylethylbenzylamine, dibenzylethylenediamine, ethanolamine, diethanolamine, 1,1,1-trishydroxymethylaminomethane, polyhydroxyalkylamine and N-methylglucosamine; inorganic acids that can form the inorganic acid addition salts include hydrochloric acid, hydrobromic acid, sulfuric acid, nitric acid and phosphoric acid; organic acids that can form the organic acid addition salts include p-toluenesulfonic acid, methanesulfonic acid, formic acid, trifluoroacetic acid, maleic acid, succinic acid and ethylsuccinic acid; and basic amino acids that can form the basic amino acid salts include lysine, arginine, ornithine and histidine. Among these salts, the base salts (i.e., inorganic base salts, ammonium salts, organic base salts and basic amino acid salts) mean base salts which can be formed at the carboxyl group at the 4 position of the compound (I) or base salts which can be produced when an acid group such as carboxyl or sulfo group is present in the substituent $R_3$ or in the substituent A, and the acid addition salts (i.e., inorganic acid addition salts and organic addition salts) mean acid addition salts which can be formed when a basic group, such as amino, mono-alkylamino group, di-alkylamino group, cycloalkylamino group, arylamino group, aralkylamino group, cyclic amino group or nitrogen-containing heterocyclic group, is present in the substituent $R_3$ or in the substituent A of the compound (I).

The ester derivatives of the compound (I) mean esters which can be formed by esterification of the carboxyl group(s), particularly the carboxyl group at the 4 position, which can be utilized as intermediates for synthesis and which are metabolically unstable, non toxic esters. Esters which can be utilized as the intermediates for synthesis include $C_{1-4}$alkyl, $C_{2-4}$ alkenyl, $C_{3-6}$ cycloalkyl, $C_{3-6}$ cycloalkyl -$C_{1-4}$ alkyl, $C_{6-10}$ aryl, $C_{7-12}$ aralkyl and substituted silyl esters, which may be further substituted. Examples of the alkyl group in the $C_{1-4}$ alkyl ester include methyl, ethyl, propyl, butyl or tert-butyl. Examples of the alkenyl group in the $C_{2-4}$ alkenyl ester include vinyl, allyl or isopropenyl. Examples of the cycloalkyl group in the $C_{3-6}$ cycloalkyl include cyclopropyl, cyclobutyl, cyclopentyl or cyclohexyl. Examples of the cycloalkyl-alkyl group in the $C_{3-6}$ cycloalkyl -$C_{1-4}$ alkyl ester include cyclopropylmethyl or cyclohexylmethyl. An example of the aryl group in the $C_{6-10}$ aryl ester is phenyl. Examples of the aralkyl group in the $C_{7-12}$aralkyl ester include benzyl or phenethyl. Examples of the substituted silyl group in the substituted silyl ester include trimethylsilyl or tertbutyldimethylsilyl. The alkyl in the aralkyl ester also may be substituted by other one or two $C_{6-10}$ aryl groups, to form for example benzhydryl ester or trityl ester.

The metabolically unstable, non-toxic ester means an orally administerable ester, which is conveniently adopted from those which have been already established in the field of penicillin and cephalosporin. Examples of such metabolically unstable, non-toxic esters include $C_{1-5}$ alkanoyloxymethyl ester, $C_{1-5}$ alkanoyloxyethyl ester, $C_{1-6}$ alkoxy-$C_{1-4}$ alkyl ester or 1-($C_{1-6}$ alkoxycarbonyloxy) $C_{1-6}$ alkyl ester, concretely such as acetoxymethyl ester, 1-acetoxyethyl ester, 1-acetoxybutyl ester, 2-acetoxyethyl ester, pivaloyloxymethyl ester, methoxymethyl ester, ethoxymethyl ester, isopropoxymethyl ester, 1-methoxyethyl ester, 1-ethoxyethyl ester, methylthiomethyl ester, ethylthiomethyl ester, ethoxycarbonyloxymethyl ester, 1-(ethoxycarbonyl)ethyl ester, tert-butoxycarbonyloxymethyl ester, 1-(tert-butoxycarbonyloxy)ethyl ester, 1-[(2-methyl-2-methoxypropionyloxy) carbonyloxy]ethyl ester and 1-(cyclohexyloxycarbonyloxy) ethyl ester. This invention includes, in addition to the ester derivatives described above, pharmaceutically acceptable compounds which are able to be converted in to the compound (I) in the organism.

The compound (I) of this invention possesses a broad spectrum of antibacterial activity and can be used for prevention and treatment of various diseases due to pathogenic bacteria in man and animals, such as a respiratory tract and urinary tract infection. The antibacterial spectrum of the compound (I) is characterized by;

(1) is a very high activity against many kinds of Gram-negative bacteria,

(2) a very high activity against Gram-positive bacteria, such as Staphylococcus aureus, Corynebacterium diphtheriae,

(3) a remarkable antibacterial action against Pseudomonas aeruginosa which is not sensitive to the usual treatment with an antibiotic agent of cephalosporin series, and

(4) a high activity against many $\beta$-lactamase-producing Gram-negative bacteria, such as the genus Escherichia, the genus Enterobacter, the genus Serratia or the genus Proteus.

The antimicrobial compound (I) of this invention is also characterized by an excellent stability, high blood level, long duration of the effect and remarkable distribution in the tissues.

Processes for preparing the cephem compound (I) of this invention, or a salt or ester thereof are in detail described in the following. The compound (I) can be prepared by three kinds of processes (1) -(3) which are known or conventional as reactions per se. That is, the compound (I) can be prepared by

(1) reacting a cephem compound of the general formula:

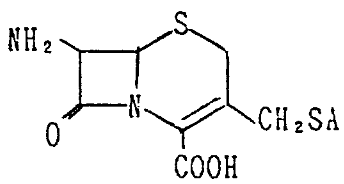

[II]

, wherein the symbol has the same meaning as described above, or a salt or ester thereof, with a carboxylic acid of the general formula.

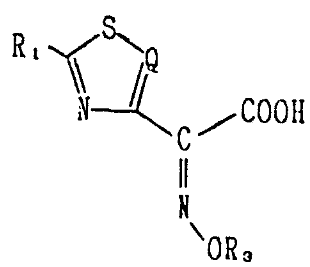

[III]

, wherein the symbols have the same meaning as described above, or a salt or reactive derivative thereof;

(2) reacting a cephem compound of the general formula

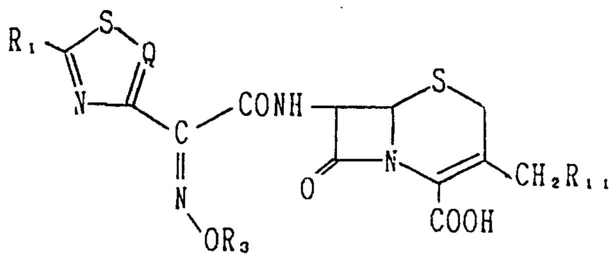

[IV]

, wherein $R_{11}$ is a hydroxy, acyloxy, carbamoyloxy or substituted carbamoyloxy group or a halogen atom and other symbols have the same meaning as described above, or a salt or ester thereof, with a thiol compound of the formula ASH in which A has the same meaning as described above, or a salt thereof; or

(3) reacting a cephem compound of the general formula:

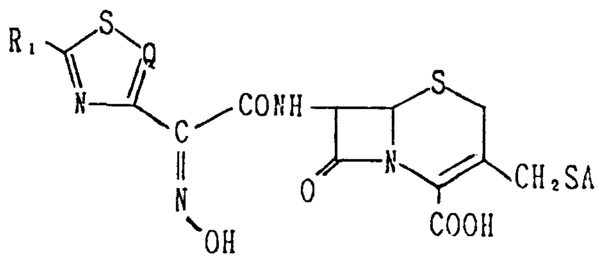

[V]

, wherein the symbols have the same meaning as described above, or a salt or ester thereof, with a compound of the general formula $R_3'OH$ in which $R_3'$ is a hydrocarbon residue which may be substituted, or a reactive derivative thereof, and then if needed, removing the protecting group.

The compound (I) in which Q is C-$R_2$ group can be also prepared by the following process (4), i.e., (4) reacting a cephem compound of the general formula:

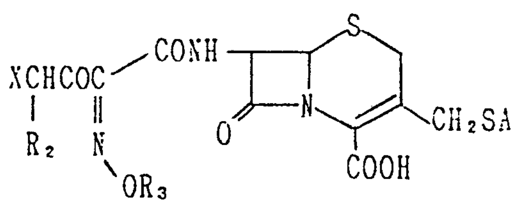

[VI]

, wherein X is a halogen or a group $R_6SO_2O$, $R_6$ is a lower alkyl group or a phenyl group which may be substituted, and other symbols have the same meaning as described above,

, or a salt or ester thereof, with a compound of the general formula $R_1C(=S)NH_2$ in which R is the same meaning as described above, and then if needed, removing the protecting group.

The processes (1) -(4), methods for removing protecting group and a purification method for the compound (I) are explained in order.

Process (1):

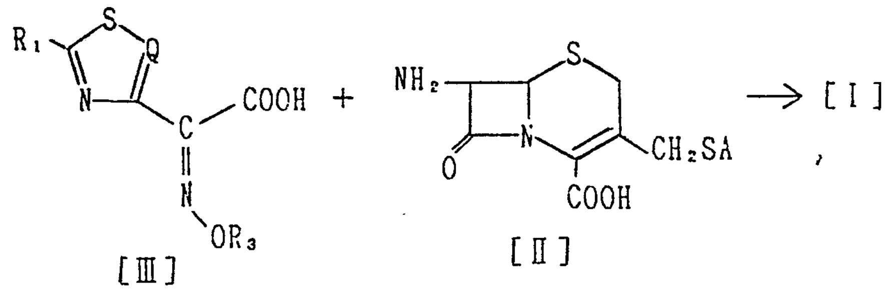

This process is the acylation of the 7-amino compound (II) with the carboxylic acid (II) or a reactive derivative thereof. In this process, the carboxylic acid (III) may be used in the free form or in the form of a salt or reactive derivative thereof, as the acylating agent for 7-amino group of the 7-amino compound (II). That is, the free acid (III), or its inorganic or organic salt, or its reactive derivative such as acid halide, acid azide, acid anhydride, mixed acid anhydride, active amide, active ester or active thioester is used for the acylation. Examples of the inorganic salt include alkalimetal salts, such as sodium or potassium salt or alkali earth metal salts such as calcium salt. Examples of the organic salt include trimethylamine salt, triethylamine salt, tert-butyldimethylamine salt, dibenzylmethylamine salt, benzyldimethylamine salt, N,N-dimethylaniline salt, pyridine salt or quinoline salt. Examples of the acid halide include acid chloride or acid bromide. Examples of the mixed acid anhydride include a mixed acid anhydride with a mono-$C_{1-4}$ alkyl carbonic acid such as a mixed acid anhydride of the free acid (III) with monomethyl carbonic acid, monoethyl carbonic acid, monoisopropyl carbonic acid, monoisobutyl carbonic acid, mono-tert-butyl carbonic acid, monobenzyl carbonic acid, mono-(p-nitrobenzyl)carbonic acid or monoallyl carbonic acid; a mixed acid anhydride with a $C_{1-6}$ aliphatic carboxylic acid such as a mixed acid anhydride of the free acid - (III) with acetic acid, cyanoacetic acid, propionic acid, butyric acid, isobutyric acid, valeric acid, isovaleric acid, pivalic acid, trifluoroacetic acid, trichloroacetic acid or acetoacetic acid; $C_{7-11}$ aromatic carboxylic acid mixed anhydride such as a mixed acid anhydride of the free acid (III) with benzoic acid, p-toluic acid or p-chlorobenzoic acid; a mixed acid anhydride with an acid such as a mixed acid anhydride of the free acid - (III) with methane sulfonic acid, ethane sulfonic acid, benzene sulfonic acid or p-toluene sulfonic acid. Examples of the active amide include an amide with a nitrogen containing heterocyclic compound such as an acid amid amide of the free acid (III) with pyrazole, imidazole or benzotriazole, which may be sub stituted by $C_{1-4}$ alkyl, $C_{1-6}$ alkoxy, halogen, oxo, thioxo or $C_{1-6}$ alkylthio. As the active ester, any of those that can be used for this purpose in the field of synthesis of $\beta$-lactams and peptides are applicable including, in addition to the organic phosphoric esters (e.g., diethoxyphosphoric or diphenoxyphosporic ester), a p-nitrophenyl, 2,4-dinitrophenyl, cyanomethyl, pentachlorophenyl, N-hydroxysuccinimido, N-hydroxyphthalimido, 1-hydroxybenzotriazole, 6-chloro-1-hydroxybenzotriazole or 1-hydroxy-1H-2-pyridone ester. Examples of the active thio ester include the ester with an aromatic heterocyclic thiol compound (e.g., a 2-pyridylthio or 2-benzothiazolylthio ester, and these heterocyclic rings may be substituted by the $C_{1-6}$ alkyl group, $C_{1-6}$ alkoxy group, halogen and $C_{1-6}$ alkylthio group described above).

The 7-amino compound (II) is used in the free form or in the form of a salt or ester thereof. Examples of the salt of the 7-amino compound (II) include inorganic base salts, ammonium salts, organic base salts, inorganic acid addition salts and organic acid addition salts. The inorganic base salts include alkali metal salts (e.g., sodium or potassium salt), and alkaline earth metal salts (e.g., calcium salt), and the organic base salts include trimethylamine, triethylamine, tert-butyldimethylamine, dibenzylmethylamine, benzyldimethylamine, N,N-dimethylaniline, pyridine or quinoline salt, and the inorganic acid addition salts include hydrochloride, hydrobromide, sulfate, nitrate or phosphate, and the organic acid addition salts include formate, acetate, trifluoroacetate, methanesulfonate and p-toluenesulfonate. As the ester of the 7-amino compound (II), the esters described above as the ester derivative of the compound (I) are applicable. That is, the esters include $C_{1-4}$ alkyl, $C_{2-4}$ alkenyl, $C_{3-6}$ cycloalkyl, $C_{3-6}$ cycloalkyl-$C_{1-4}$ alkyl, $C_{6-10}$ aryl, $C_{7-12}$ aralkyl, $C_{1-5}$ alkanoyloxymethyl or $C_{1-5}$ alkanoyloxyethyl ester, and the corresponding esters which are substituted by hydroxy, $C_{1-6}$ alkoxy, halogen, nitro, cyano, mercapto, $C_{1-6}$ alkylthio, oxo or thioxo group.

The starting compound (II) and salt or ester thereof, and the starting material (III) and reactive derivative thereof can be easily prepared by a known method (e.g., U.S.P. 4,358,448; EP-150507A; U.S.P. 3,907,786; U.S.P. 4,463,003) or one analogous thereto.

The reactive derivative of the compound (III) may be reacted with the compound (II), after isolation from the reaction mixture, or the reaction mixture containing a reactive derivative of the compound (III) may be, without being isolated, reacted with the compound (II). When the carboxylic acid (III) is used in the form of a free acid or a salt, an appropriate condensing agent is used. Examples of the condensing agent include a N,N'-disubstituted carbodiimide, such as N,N'-dicyclohexylcarbodiimide, an azolide, such as N,N'-carbonyldiimidazole and N,N'-thiocarbonyldi imidazole, a dehydrating agent, such as N-ethoxycarbonyl-2-ethoxy-1,2-dihydroquinoline, phosphorus oxychloride and alkoxyacetylene and a 2-halogenopyridinium salt, such as 2-chloropyridinium methyl iodide or 2-fluoropyridinium methyl iodide. When any of these condensing agents is used, the reaction is supposed to proceed through a reactive derivative of the carboxylic acid (III). The reaction is generally carried out in a solvent and a solvent which does not interfere with the reaction is selected appropriately. Examples of the solvents include ethers, such as dioxane, tertrahydrofuran, diethyl ether, tert-butyl methyl ether, diisopropyl ether or ethyleneglycol dimethyl ether; esters, such as ethyl formate,ethyl acetate or n-butyl acetate, halogenated hydrocarbons, such as dichloromethane, chloroform, carbon tetrachloride, trichlene or 1,2-dichloroethane; hydrocarbons, such as n-hexane, benzene or toluene; amides, such as formamide, N,N-dimethylformamide or N,N-dimethylacetamide; ketones, such as acetone, methylethylketone or methylisobutylketone; nitriles, such as acetonitrile or propionitrile; dimethyl sulfoxide, sulfolane, hexamethylphosphoramide and water, which are used alone or as a mixture thereof.

The acylating agent (III) is usually used in an amount of 1 to 5 moles, preferably 1 to 2 moles, per 1 mole of the compound (II). The reaction is conducted at -80 to 80°C, preferably at -40 to 50°C, and most desirably at -30 to 30°C. The reaction time varies depending on the species of the compounds (II) and (III), the kind of the solvent (also the mixing ratio if a mixed solvent is used), the reaction temperature, etc., being usually 1 minute to 72 hours, preferably 15 minutes to 3 hours.

When an acid halide is used as the acylating agent, the reaction may be conducted in the presence of an acid-binding agent to remove the liberated hydrogen halide out of the reaction system. Examples of the such acid-binding agent include inorganic bases, such as sodium carbonate, potassium carbonate, calcium carbonate or sodium hydrogen carbonate; tertiary amines, such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine or N-methylmorpholine and alkyleneoxides, such as propyleneoxide and epichlorohydrin.

Process (2)

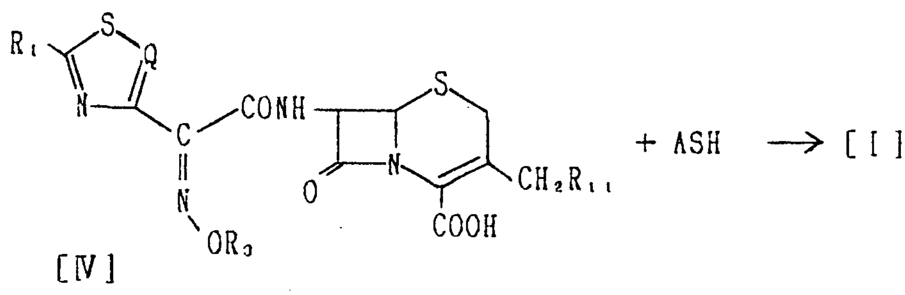


[IV]

This process comprises reacting the cephem (IV) with a thiol compound ASH where the cephem compound (I) is synthesized by nucleophilic substitution.

$R_{11}$ in the formula (IV) represents a hydroxy, acyloxy, carbamoyloxy or substituted carbamoyloxy group or a halogen atom. The acyloxy group includes $C_{2-5}$ alkanoyloxy or $C_{6-10}$aryl-acyloxy group which may be substitued. Examples of the $C_{2-5}$ alkanoyloxy group which may be substituted include acetoxy, propionyloxy, butyryloxy, pivaloyloxy, 3-oxobutyryloxo, 4-chloro-3-oxybutyryloxy, 3-carboxypropionyloxy, 4-carboxybutyryloxy or 3-ethoxycarbamoylpropionyloxy. Examples of the $C_{6-10}$ aryl-acyloxy group which may be substituted include o-carboxybenzoyloxy, o-(ethoxycarbonylcarbamoyl)benzoyloxy or o-(ethoxycarbonylsulfamoyl)benzoyloxy. Examples of the substituted carbamoyloxy group include methylcarbamoyloxy or N,N-dimethylcarbamoyloxy. Examples of the halogen atom include chlorine, bromine or iodine.

The compound (IV) may be employed in the free form or in the form of a salt or ester thereof. As the salt and ester of the compound (IV), those of the compound (II) stated in Process (1) are also here applicable. The compound (IV) or a salt or ester thereof, and the thiol compound ASH or salt thereof can be easily prepared by using a known method (e.g., U.S.P. 4,358,448; EP-150507A) or one analogous thereto. The thiol compound ASH may be employed in the form of salt. Examples of the salt of the thiol compound ASH include alkali metal salts such as sodium or potassium salt, or alkali earth metal salts such as calcium salt.

The nucleophilic substitution of the thiol compound ASH or salt thereof to the compound belongs to a reaction known per se and is usually conducted in a solvent. As the solvent employed in this reaction, ones employed in Process (1), such as ethers, esters, helogenated hydrocarbons, hydrocarbons, amides, ketones, nitriles or water are here applicable. In addition, alcohols such as methanol, ethanol, propanol, isopropanol, ethylene glucol or 2-methyoxyethanol are also usable.

(2-1) : When $R_{11}$ is acyloxy, carbamolyoxy or substituted carbamoyloxy group

More preferred solvent is water, or a mixture of a water-miscible organic solvent and water. The preferred organic solvent is acetone, methylethylketone or acetonitrile. The thiol compound ASH or salt thereof is usually used in an amount of 1 to 5 moles, preferably 1 to 3 moles, per 1 moe of the compound - (IV). The reaction is conducted at a temperature range of 10 -100°C, peferably 30 -80°C. The reaction time varies depending upon the species of the compound (IV) and the compound ASH, the kind of the solvent (a mixing ratio in case of the mixed solvent) and the reaction temperature and is usually for 10 minutes to 5 days, preferably 10 minutes to 5 hours. The reaction is advantageously conducted at a pH of 2 to 8, peferably at a nuetral region, i.e., at pH 5 to 8.

(2-2) : When $R_{11}$ is hydroxy group

The reaction is conducted in the presence of an organic phosphorus compound according to the method described, for example, in Japanese Laid-Open Patent Application No. 43979/83.

Examples of the organic phosphorus compound include o-phenylenephosphorochloridate, o-phenylenephosphorofluoridate, methyl o-phenylenephosphate, ethyl o-phenylenephosphate, propyl o-phenylenephosphate, isopropyl o-phenylenephosphate, n-butyl o-phenylenephosphate, isobutyl o-phenylenephosphate, sec-butyl o-phenylenephosphate, cyclohexyl o-phenylenephosphate, phenyl o-phenylenephosphate, p-chlorophenyl o-phenylenephosphate, p-acetylphenyl o-phenylenephosphate, 2-chloroethyl o-phenylenephosphate, 2,2,2-trichloroethyl o-phenylenephosphate, ethoxycarbonylmethyl o-phenylenephosphate, carbamoylmethyl o-phenylenephosphate, 2-cyanoethyl o-phenylenephosphate, 2-methylsulfonylethyl o-phenylenephosphate, benzyl o-phenylenephosphate, 1,1-dimethyl-2-propenyl o-phenylenephosphate, 2-propenyl o-phenylenephosphate, 3-methyl-2-butenyl o-phenylenephosphate, 2-thienylmethyl o-phenylenephosphate, 2-furfurylmethyl o-phenylenephosphate, bis-o-phenylenepyrophosphate, 2-phenyl-1,3,2-benzodioxaphosphole-2-oxide, 2-(p-chlorophenyl)-1,3,2-benzodioxaphosphole-2-oxide, 2-butyl-1,3,2-benzodioxaphosphole-2-oxide, 2-anilino-1,3,2-benzodioxaphosphole-2-oxide, 2-phenylthio-1,3,2-benzodioxaphosphole-2-oxide, 2-methoxy-5-methyl-1,3,2-benzodioxaphosphole-2-oxide, 2-chloro-5-ethoxycarbonyl-1,3,2-benzodioxaphosphole-2-oxide, 2-methoxy-5-ethoxycarbonyl-1,3,2-benzodioxaphosphole-2-oxide, 5-ethoxycarbonyl-2-phenyl-1,3,2-benzodioxaphosphole-2-oxide, 2,5-dichloro-1,3,2-benzodioxaphosphole-2-oxide, 4-chloro-2-methoxy-1,3,2-benzodioxaphosphole-2-oxide, 2-methoxy-4-methyl-1,3,2-benzodioxaphosphole-2-oxide, 2,3-naphthalenemethylphosphate, 5,6-dimethyl-2-methoxy-1,3,2-benzodioxaphosphole-2-oxide, 2,2-dihydro-4.5,6,7-tetrachloro-2,2,2-trimethoxy-1,3,2-benzodioxaphosphole, 2,2-dihydro-4,5,6,7-tetrachloro-2,2,2-triphenoxy-1,3,2-benzodioxaphosphole, 2,3-dihydro-2,2-ethylenedioxy-2-methoxy-1,3,2-benzodioxaphosphole, 2,2-dihydro-2-benzyl-2,2-dimethoxy-1,3,2-benzodioxaphosphole, 2,2-dihydro-4,5-benzo-2,2,2-trimethoxy-1,3,2-benzodioxaphophole, 2,2-dihydro-2,2,2-triphenoxy-1,3,2-benzodioxaphosphole, 2,2-dihydro-2,2-(o-phenylenedioxy)-2-phenoxy-1,3,2-benzodioxaphosphole, 2-chloro-2,2-dihydro-2,2-(o-phenylenedioxy)-1,3,2-benzodioxaphosphole, 2,2-dihydro-2-methoxy-2,2-(o-phenylenedioxy)-1,3,2-benzodioxaphosphole, 2,2-dihydro-2,2,2-trichloro-1,3,2-benzodioxaphosphole, 9,10-phenanthrenedioxytrimethoxyphosphorus, o-phenylene phosphochloridite, o-phenylenephosphorobromidite, o-phenylenephosphorofluoridite, methyl o-phenylenephosphite, butyl o-phenylenephosphite, methoxycarbonylmethyl o-phenylenephosphite, phenyl o-phenylenephosphite, p-chloro (or p-nitro)phenyl o-phenylenephosphite, 2-phenyl-1,3,2-benzodioxaphosphole, bis-o-phenylenepyrophosphite, 2-methoxy-5-methyl-1,3,2-benzodioxaphosphole, 5-acetyl-2-phenoxy-1,3,2-benzodioxaphosphole, 9,10-phenanthrenephosphorochloridite, 2-chloro-4-methyl-1,3,2-benzodioxaphosphole, 5-ethoxycarbonyl-2-phenyl-1,3,2-benzodioxaphosphole, 2-chloro-2-thioxo-1,3,2-benzodioxaphosphole, 2-phenoxy-2-oxo-1,3,2-benzodiazaphosphole, 2-phenoxy-1,3,2-benzodioxaazaphosphole, 2,2-dihydro-2-oxo-2-methoxy-4,5-dimethyl-1,3,2-dioxaphosphole, 2,2-dihydro-2-oxo-2-chloro-4,5-dimethyl-1,3,2-dioxaphosphole, 2,2-dihydro-2-oxo-2-(1-imidazolyl)-4,5-dimethyl-1,3,2-dioxaphosphole, 2,2-dihydro-2,2-ethylenedioxy-2-methoxy-4,5-dimethyl-1,3,2-dioxaphosphole, 2,2-dihydro-2,2-dimethoxy-2-phenoxy-4,5-dimethyl-1,3,2-dioxaphosphole, 2,2-dihydro-2,2,2-trimethyoxy-4,5-dimethyl-1,3,2-dioxaphosphole, 2,2-dihydro-2,2,2-triphenoxy-4,5-dimethyl-1,3,2-dioxaphosphole, 2,2-dihydro-2,2,2-triethoxy-4,5-diphenyl-1,3,2-dioxaphosphole, 2,2-dihydro2,2,2-trimethoxy-4,5-diphenyl-1,3,2-dioxaphosphole, 2,2-dihydro-2-oxo-2-methoxy-4,5-diphenyl-1,3,2-dioxaphosphole, 2,2-dihydro-2,2,2-trimethoxy-1,3,2-dioxaphosphole, 2,2-dihydro-2,2,2-trimethoxy-4-phenyl-1,3,2-dioxaphosphole, 2,2-dihydro-2,2,2-trimethoxy-4-methyl-1,3,2-dioxaphosphole, 2,2-dihydro-2,2,2-trimethoxy-4-methyl-5-phenylcarbamoyl-1,3,2-dioxaphosphole, 2,2,4,5,6,7-hexahydro-2,2,2-trimethoxy-1,3,2-benzodioxaphosphole, 2,2'-oxybis(4,5-dimethyl-2,2-dihydro-1,3,2-dioxaphosphole), and 2,2'-oxybis(4,5-dimethyl-2,2-dihydro-1,3,2-dioxaphosphole-2-oxide).

The reaction is usually carried out in a solvent which does not interfere with the reaction. Preferred examples of the solvent include the ethers, the esters, the halogenated hydrocarbons, the hydrocarbons, the amides, the ketones and the nitriles described above, which may be used alone or in a mixture thereof. Especially dichloromethane, acetonitrile, formamide, a mixtrue of formamide and acetonitrile, and a mixture of dichloromethane and acetonitrile bring about a good result. The amount of the thiol compound ASH or a salt thereof and the amount of the organic phosphorus compound are 1 to 5 moles and 1 to 10 moles, per 1 mole of the compound (IV), respectively, preferably 1 to 3 moles and 1 to 6 moles, respectively. The reaction is conducted at -80 to 50°C, preferably at -40 to 40°C. The reaction time is usually 1 minute to 15 hours, peferably 5 minutes to 2 hours. An organic base may be added to the reaction system. Examples of the organic base include amines, such as triethylamine, tri(n-butyl)amine, di(n-butyl)amine, diisobutylamine, dicyclohexylamine, diisobutylamine, dicyclohexylamine and 2,6-lutidine. The base is preferably used in an amount of 1 to 5 moles per 1 mole of the compound (IV).

(2-3) : When $R^5$ is halogen:

Preferred solvents are the ethers, esters, hydrocarbon halides, hydrocarbons, amides, ketones, nitriles, alcohols and water described above. The thiol compound ASH or a salt thereof is usually used in an amount of 1 to 5 moles, preferably 1 to 3 moles per 1 mole of the compound (IV). The reaction is conducted at 0 to 80°C, preferably at 20 to 60°C. The reaction time is usually 30 minutes to 15 hours, preferably 1 to 5 hours. The reaction can be conducted in the presence of an acid binding agent to accelerate the reaction. As the acid binding agent use is made of the acid-binding agents described in Process (1), such as inorganic bases, tertiary amines or alkylene oxides. The halogen represented by $R^5$ is chlorine, bromine or iodine, among which iodine is preferable. The compound (IV) in which $R^5$ is iodine can be produced easily, for example, by the method described in Japanese Laid-Open Patent Application No. 57390/83 or by a method analogous thereto.

Process (3):

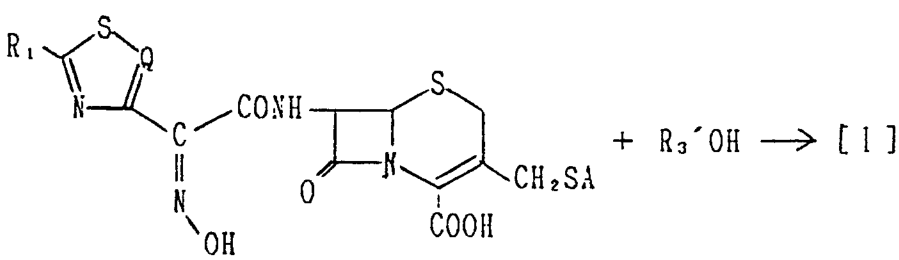

(V)

This process consists in synthesis of the compound (I) by reacting the hydroxyimino compound (V) with a compound of the general formula $R_3'OH$ or a reactive derivative thereof, which is a conventional etherification. $R_3'$ represents a hydrocarbon residue which may be substituted. The hydrocarbon residue which may be substituted as stated in $R_3$ is here applicable.

$R_3'OH$ may be used in a free form or as a reactive derivative. The reactive derivative of $R_3'OH$ means the derivative of $R_3'OH$ having a group which can be removed together with the hydrogen atom of the hydroxyimino compound (V), that is, a compound of the general formula $R^{3''}Y$. The group Y which is removed together with the hydrogen atom is halogen, sulfo or a mono-substituted sulfonyloxy. Examples of the halogen atom include chlorine, bromine and iodine. Examples of the mono-substituted sulfonyloxy group include $C_{1-4}$ alkylsulfonyloxy or $C_{6-10}$ arylsulfonyloxy, such as methanesulfonyloxy, ethanesulfonyloxy, benzenesulfonyloxy or p-toluenesulfonyloxy. Especially when C $_{1-4}$ alkyl ester derivatives of the compound - (V) are produced, not only the reactive derivative described above, but also $C_{1-4}$ diazoalkane such as diazomethane and diazoethane and di-$C_{1-4}$ alkyl sulfate such as dimethyl sulfate and diethyl sulfate may be used.

(3-1) : When $R^{3'}OH$ is used:

The compound (I) is synthesized by reacting the hydroxyimino compound (V) with the compound $R^{3'}OH$ in the presence of an appropriate dehydrating agent. Examples of the dehydrating agent include phosphorus oxychloride, thionyl chloride, dialkyl azodicarbonate (usually used in the presence of phosphine) and N,N'-dicyclolohexylcarbodiimide, preferably diethyl azodicarbonate in the presence of triphenylphosphine. The reaction using diethyl azodicarbonate in the presence of triphenylphosphine is usually carried out in an anhydrous solvent, such as the ethers or hydrocarbons described above. One to 1.5 moles each of the compound $R^{3'}OH$, ethyl azodicarbonate and triphenylphosphine are used per 1 mole of the hydroxyimino compound (V). The reaction takes 1 to 4 days at 0 to 50°C.

(3-2) : When $R_3'Y$ is used:

The reaction of the compound $R_3'Y$ with the hydroxyimino compound (V) is a usual ether-formation reaction, which is carried out in a solvent. The solvents are the ethers, esters, halogenated hydrocarbons, hydrocarbons, amides, ketones, nitriles, alcohols, and water as described in the Process (1), which are used alone or in a mixture thereof, preferably a mixed solvent consisting of a solvent miscible with water and water (e.g., aqueous methanol, aqueous ethanol, aqueous acetone or aqueous dimethylsulfoxide). This reaction may be carried out smoothly in the presence of an appropriate base. Examples of the base include inorganic bases, such as alkali metal salts exemplified by sodium carbonate, sodium hydrogen carbonate or potassium carbonate and alkali metal hydroxides exemplified by sodium hydroxide or potassium hydroxide. This reaction may be conducted in a buffer solution of pH 7.5 to 8.5 The reagent $R^{3'}Y$ is used in an amount of 1 to 5 moles, peferably 1 to 3 moles, per 1 mole of the starting compound (V) and the base is used in an amount of 1 to 10 moles, preferably 1 to 5 moles, per 1 mole of the starting compound (V). The reaction temperature is -30 to 100°C, preferably 0 to 80°C. The reaction time is 10 minutes to 15 hours, preferably 30 minutes to 5 hours.

(3-3) : When $C_{1-4}$ diazoalkane is used:

The reaction is usually conducted in a solvent. As the solvent, ⁺he ethers and hydrocarbons described above are used. The reaction proceeds when a solution of a diazoalkane compound is added to a solution of the hydroxyimino compound (V). One to 10 moles, preferably 1 to 5 moles of the reagent is used per 1 mole of the compound (V). The reaction is carried out at a relatively low temperature, i.e., at -50 to 20°C, preferably at -30 to 0°C. The reaction time is 1 minute to 5 hours, preferably 10 minutes to 1 hour.

(3-4) :When di-$C_{1-4}$ alkyl sulfate is used:

The reaction is usually conducted in water or in a mixed solvent consisting of a solvent miscible with water and water. Examples of the mixed solvents are also here the aqueous solvents described in the Process (3-2). This reaction is usually conducted in the presence of an inorganic base, such as alkali metal hydroxides exemplified by sodium hydroxide or potassium hydroxide. Per one mole of the compound (V), 0.5 to 10 moles, preferably 1 to 2 moles of the reagent are used. The reaction temperature is 20 to 100°C, preferably 50 to 100°C. The reaction time is 10 minutes to 5 hours, preferably 30 minutes to 3 hours.

Process (4) : The reaction is written by the following formula,

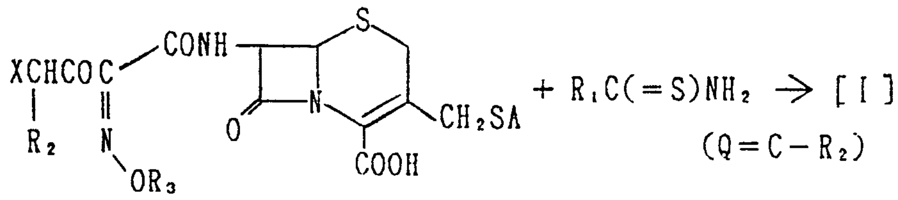


[VI]

This method consists in synthesis of the object compound (I) ($Q=C-R_2$) by reacting the compound (VI) with thiourea or a thiourea derivative of the general formula $R_1C(=S)NH_2$. The compound (VI) may be used in a free form or in the form of a salt or ester thereof. In the compound (VI), X is a halogen atom or a group of $R_6CO_2O$. The halogen atom is chlorine, bromine or iodine. $R_6$ represents a lower alkyl group or a phenyl group which may be substituted. Examples of the lower alkyl group include $C_{1-4}$ alkyl such as methyl, ethyl, or propyl. Examples of the phenyl group which may be substituted include p-methylphenyl, p-methoxyphenyl, p-chlorophenyl or p-nitrophenyl. Examples of the salt of the compound (VI) are the salt of the 7-amino compound (II) described in the Process (1) (for example, inorganic base salts, ammonium salts, organic base salts, inorganic acid addition salts or organic acid addition salts). Examples of the ester of the compound (IV) are the ester of the 7-amino compound (II) described in the Process (1) for example, the $C_{1-4}$ alkyl, $C_{2-4}$ alkenyl, $C_{3-6}$ cycloalkyl, $C_{3-6}$ cycloalkyl-$C_{1-4}$ alkyl, $C_{6-10}$ aryl, $C_{7-12}$ aralkyl, $C_{1-5}$ alkanoyloxymethyl or $C_{1-5}$ alkanoyloxyethyl ester. The starting compound (VI) can be easily synthesized by reacting a compound of the general formula:

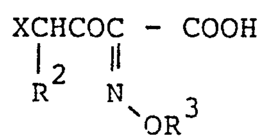

wherein the symbols are of the same meanings as defined above, or a salt or reactive derivative thereof, with the 7-amino compound (II) or a salt or ester thereof described above, according to the method described in the Process (1). The compound of the general formula:

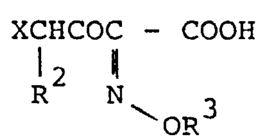

or a reactive derivative thereof can be easily prepared by a per se known method or by one analogous thereto. The reaction of the compound (VI) with the compound $R'C(S=)NH_2$ is usually carried out in a solvent. As the solvent, ethers, such as dioxane, tetrahydrofuran or diethylether, alcohols, such as methanol,

ethanol or n-propanol, and amides, such as dimethylformamide or dimethylacetamide are used. The amount of thiourea or a derivative thereof $R^1C(S=)NH_2$ is usually 1 to 5 moles, preferably 1 to 3 moles, per 1 mole of the compound (VI). The reaction may be conducted at 0 to 100°C, preferably 20 to 60°C. The reaction time is usually 30 minutes to 15 hours, preferably 1 to 5 hours.

Accordingly to the above mentioend Process (1) -(4), the compound (I) is sometimes obtained as a mixture of the syn(Z)-and anti(E)-isomers. The desired syn-isomer may be isolated from the mixture by a per se known method or one analogous thereto, such as fractionation by taking advantage of the difference in solubility or crystalinity, chromatographic separation and separation by taking advantage of the difference in rate of hydrolysis of the ester derivatives.

Method of Removal of the Protective Group:

As described above, amino-protective groups have been extensively investigated and the methods of protection have been established in the fields of synthesis of $\beta$-lactams and peptides. Also the methods of removal of amino-protective groups have been established, and the known procedures of removal of the protective groups are applicable also to the present invention. For example, a monohalogenoacetyl group -(such as chloroacetyl or bromoacetyl) can be removed with thiourea, an alkoxycarbonyl group (such as methoxycarbonyl, ethoxycarbonyl or tert-butoxycarbonyl) with an acid (such as hydrochloric acid), an aralkyloxycarbonyl group (such as benzyloxycarbonyl, p-methylbenzyloxycarbonyl, or p-nitrobenzyloxycarbonyl) by catalytic reduction, and a 2,2,2-trichloroethoxycarbonyl group with zinc and an acid (such as acetic acid). When the compound (I) is esterified, the ester residue can be also removed by a per se known procedure or one analogous thereto. For example, the 2-methylsulfonyl ethyl ester group can be removed with an alkali, an aralkyl ester group (such as a benzyl, p-methoxybenzyl, or p-nitrobenzyl ester) with an acid (such as trifluoroacetic acid) or by catalytic reduction, the 2,2,2-trichloroethyl ester group with zinc and an acid (such as acetic acid), and a silyl ester group (such as a trimethylsilyl, or tert-butyl dimethylsilyl ester) with water alone.

Method of Purification of the Compound (I):

The compound (I) producėd in the reaction mixture by the methods described in detail in the Processes (1) to (4), followed by, if necessary, the removal of the protective group according to the method described above, can be isolated and purified by a known procedure, such as extraction, column chromatography, precipitation or/and recrystallization. When the thus isolated compound (I) is not a salt or ester, it can be converted into a desired pharmaceutically acceptable salt or a metabolically unstable, non-toxic ester by known procedure or one analogous thereto.

The sulfoxide (n=1) of the cephem compounds (I) is obtained by oxidation of the compounds [(I), n=0). Such oxidation is conventional. Oxidizing agents suitable for the oxidation of the sulfur atom in the cephem ring include oxygen, peracids, hydroperoxides and hydrogen peroxide, and the peracids can be easily produced by mix. ing an acid with a peroxide during the reaction. As the peracids, peracetic acid, perbenzoic acid and p-chloroperbenzoic acid are frequently used. The reaction is usually conducted in a solvent. Examples of the solvent used for this reaction include ethers, such as dioxane or tetrahydrofuran, halogenated hydrocarbons, such as dichloromethane, chloroform or chlorobenzene, organic acids, such as formic acid, acetic acid or trifluoroacetic acid, and amides, such as dimethylformamide or dimethylacetamide. The reaction is conducted at -20 to 80°C, preferably at a temperature as low as possible, desirably -20 to 20°C. It is generally known that a sulfoxide of S-configuration is predominantly produced by oxidation of a cephem compound [(I), n=o). R-and S-sulfoxides are fractionated by taking advantage of the difference in their solubility or mobility in chromatographic separation. The oxidation reaction to produce the sulfoxides described above may be conducted prior to, or subsequently to, the reactions described in the Process (1) -(4).

The compound (I) of this invention is able to be administered parenterally or orally in the form of injections, capsules, tablets, and granules, as the known penicillins and cephalosporins. The dose is 0.5 to 80 mg/day/kg body weight of a man or an animal infected with a pathogenic bacteria described above, preferably 1 to 20 mg/day/kg, given 3 or 4 times a day. The excipients used for injections include distilled water and physiological saline. For capsules, powders, granules and tablets, a known pharmaceutically

acceptable excipient (e.g., starch, lactose, sucrose, calcium carbonate or calcium phosphate), a binder (e.g., starch, gum arabic, carboxymethylcellulose, hydroxypropylcellulose or crystalline cellulose), a lubricant - (e.g., magnesium stearate or talc) and a disintegrating agent (e.g., carboxymethylcellulose, calcium or talc) are used.

A pharmaceutical composition containing the compound (I) or a salt or ester thereof can be produced by a known method. The said composition can be produced usually by mixing at least one of the compound (I) and pharmaceutically acceptable salts or esters thereof with the carriers, excipients etc., described above. The ratio of the compound (I) contained in a composition is usually 5 to 100%, preferably 20 to 100% by weight in solid compositions, such as capsules, tablets or granules, and 5 to 30% by weight in liquid compositions, such as injections.

The compound (I) or a salt or ester thereof is given in the form of injection for treatment of urinary tract infection caused by Escherichia coli. The dose in this case is 0.5 to 80 mg/day/kg body weight of a man or animal, preferably 1 to 20 mg/day/kg, given 3 or 4 times a day. Such injections can be produced easily, for example, by dissolving or suspending the compound (I) or a salt or ester thereof in physiological saline.

(Examples and Reference Examples)

This invention is illustrated in further detail in the Reference Examples and Examples, which are only examples, and do not limit this invention. Modification within the scope of this invention are permissible.

Elution in a column chromatography in the Reference Examples and Examples was conducted while monitoring with TLC (Thin Layer Chromatography). In the TLC monitoring, the TLC plate used was $BOF_{254}$ manufactured by Merck Co., the developing solvent was the same to the one used for eluting in the column chromatography, and the detection was conducted with a UV detector was employed for detection. The silica gel for the column was Kieselgel 60 manufactured by Merck Co. (West Germany), (230-400 mesh). "Sephadex" is a product of Pharmacia Fine Chemicals Co. (Sweden). XAD-II resin is a product of Rohm & Haas Co. (U.S.A.). NMR spectra were measured using tetramethylsilane as an internal or external standard with a spectrometer XL-100A (10MHz), EM360 (60 MHz), EM390 (90MHz) or $T_{60}$ (60MHz) type, and all $\delta$ values are expressed in ppm. The value shown in ( ) for a mixed solvent is a mixing ratio in volume of constituent solvents. The percentage (%) for a solution indicates the grams in 100 ml of the solution. The symbols in Reference Examples and Examples mean as follows.

s : singlet
d : doublet
t : triplet
q : quartet
ABq : AB type quartet
d.d : double doublet
m : multiplet
br. : broad
J : coupling constant
Hz : Herz
mg : milligram
g : gram
ml : milliliter
l : liter
% : percent
DMSO : dimethyl sulfoxide
$D_2O$ : deuterium oxide
$CDCl_3$ : deuterochloroform

Example 1

Sodium 7$\beta$-[2-(2-aminothiazol-4-yl)-2(Z)-methoxyiminoacetamido]-3-[(s-triazolo[4,3-b]pyridazin-3-yl)-thiomethyl]-3-cephem-4-carboxylate

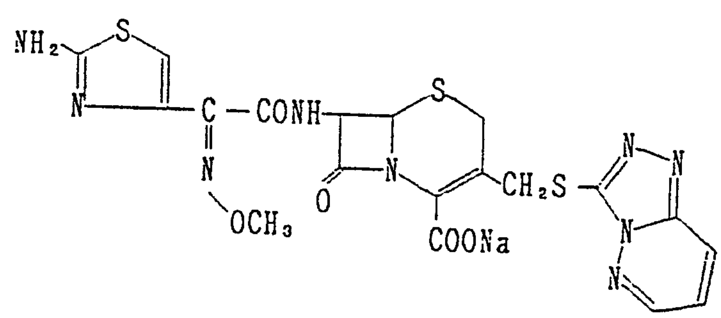

A mixture of 1.0 g of 7β-[2-(2-aminothizol-4-yl)-2(Z)-methoxyiminoacetamido]-3-(3-oxobutyryloxy)-methyl-3-cephem-4-carboxylic acid, 328 mg of sodium bicarbonate, 500 mg of 3-mercapto-s-triazol [4,3-b] pyridazine and 20 ml of water was stirred at 70 °C for 30 minutes. After the reaction was completed, the reaction mixture was subjected to chromatography on an XAD-II coloum eluting with water and 10 % ethanol in turn.

The pertinent fraction was concentrated and then lyophilized to give 0.5 g of the title compound.
Elemental analysis for $C_{18}H_{16}O_5S_3Na \bullet 2H_2O$:
Calcd.(%) : C,37.68: H,3.33: N,20.82
Found (%) : C,37.48: H,3.54: N,20.73
IR $\nu^{KBr}_{max}$ $cm^{-1}$: 1760, 1680, 1610, 1530
NMR($d_8$-DMSO) δ : 3.75(3H,s), 4.40(2H,s), 5.03(1H,d,J=5Hz), 5.63(1H,d.d,J=9Hz & 5Hz), 6.73(1H,s), 7.23-(2H,s), 7.33(1H,d.d,J=9Hz & 4.5Hz), 8.33(1H,d.d,J=9Hz & 1.5Hz), 8.65(1H,d.d,J=4.5Hz & 1.5Hz), 9.66-(1H,d,J=9Hz)

Example 2

Sodium 7β-[2-(2-aminothiazol-4-yl)-2(Z)-methoxyiminoacetamido]-3-[(s-triazolo[4,3-a] pyridin-3-yl)-thiomethyl]-3-cephem-4-carboxylate

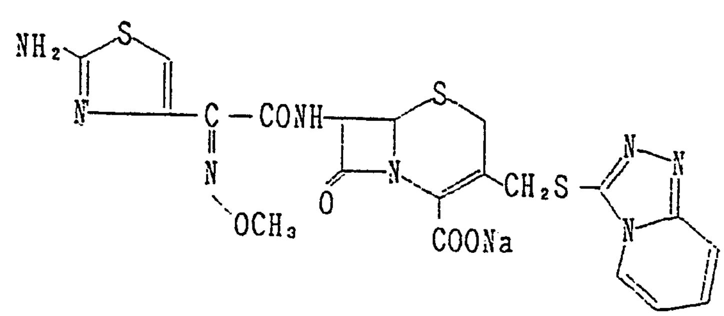

Using 7β-[2-(2-aminothiazol-4-yl)-2(Z)-methoxyiminoacetamido]-3-(3-oxobutyryloxy)methyl-3-cephem-4-carboxylic acid and 3-mercapto-s-triazolo[4,3-a]pyridine, the mixture was reacted by the same method as described in Example 1 to give the title compound.
Elemental analysis for $C_{20}H_{17}N_8O_5S_3Na \bullet 3H_2O$:
Calcd. (%) : C,38.58; H,3.72; N,18.00
Found (%) : C,38.83; H,3.83; N,17.59
IR $\nu^{KBr}_{max}$ $cm^{-1}$: 1760, 1675, 1610
NMR ($d_6$-DMSO)δ : 3.90(3H,s), 4.10 & 4.40(2H,ABq,J=12Hz), 5.00(1H,d,J=5Hz), 5.63(1H,d.d,J=9Hz & 5Hz), 6.70(1H,s), 7.00-7.50(6H,m), 7.80(1H,d,J=9Hz), 8.45(1H,d,J=6Hz), 9.53(1H,d,J=9Hz)

Example 3

Sodium 7β-[2-(2-aminothiazol-4-yl)-2(Z)-methoxyiminoacetamido]-3-[(s-triazolo[3,4-a]phthalazin-3-yl)-thiomethyl]-3-cephem-4-carboxylate

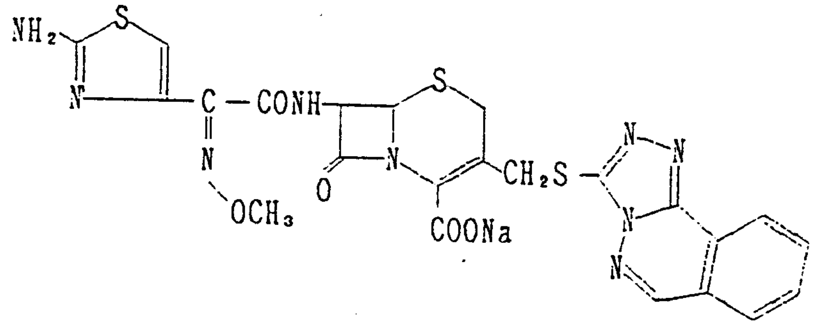

Using 7β-[2-(2-aminothiazol-4-yl)-2(Z)-methoxyiminoacetamido]-3-(3-oxobutyryloxy)methyl-3-cephem-4-carboxylic acid and 3-mercapto-s-triazolo[3,4-a]phthalazine, the mixture was reacted by the same method as described in Example 1 to give the title compound.
Elemental analysis for $C_{23}H_{18}N_9O_5S_3Na \bullet 2.5H_2O$:
Calcd. (%) : C,41.56; H,3.49; N,18.97
Found (%) : D,41.64; H,3.57; N,18.68
IR $^{KBr}_{max}$ $cm^{-1}$: 1760, 1680, 1600, 1530
NMR ($d_6$-DMSO)δ: 3.96(3H,s), 4.53(2H,s), 5.10(1H,d,J=5Hz), 5.73(1H,d.d,J=9Hz & 5Hz), 6.66(1H,s), 7.30-(2H,br.,s), 7.90-8.63(4H,m), 9.03(1H,s), 9.60(1H,d,J=9Hz)

Example 4

Sodium 7β-[2-(2-aminothiazol-4-yl)-2(Z)-methoxyiminoacetamido]-3-[(6-morpholino-s-traizolo[4,3-b]-pyridazin-3-yl)thioethyl]-3-cephem-4-carboxylate

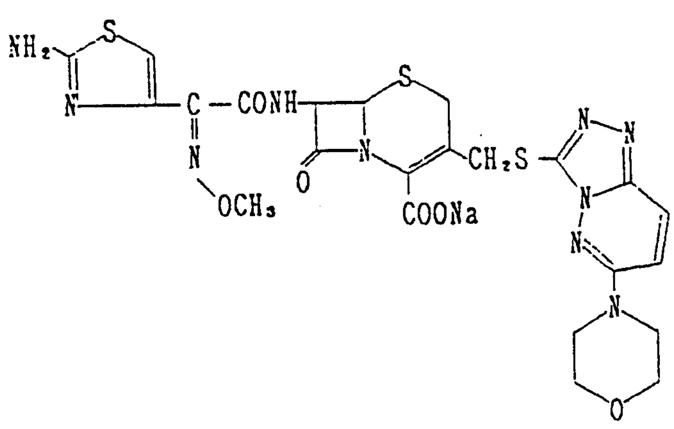

7β-[2-(2-Aiminothiazol-4-yl)-2(Z)-methoxyiminoacetamido]-3-(3-oxobutyryloxy)methyl-3-cephem-4-carboxylic acid and 3-mercapto-6-morpholino-s-triazolo[4,3-b]pyridazine were reacted by the same method as described in Example 1 to give the title compound.
Elemental analysis for $C_{23}H_{23}N_{10}O_6S_3Na \bullet 2.5H_2O$:
Calcd. (%) : C,39.47; H,4.03; N,20.02
Found (%) : C,39.57; H,4.11; N,19,79
IR $\nu^{KBr}_{max}$ $cm^{-1}$: 1760, 1670, 1610
NMR($d_6$-DMSO)δ: 3.00-3.70(10H,m), 3.75(3H,s), 4.23 (2H,s), 4.90(1H,d,J=5Hz), 5.50(1H,d.d,J=9Hz & 5Hz), 6.60(1H,s), 7.00(2H,s), 7.20(1H,d,J=10Hz), 7.90(1H, d,J=10Hz, 9.36(1H,d,J=9Hz)

Example 5

Sodium 7β-[2-(2-aminothiazol-4-yl)-2(Z)-methoxyiminoacetamido]-3-[(6-phenyl-s-triazolo[4,3-b]pyridazin-3-yl)thiomethyl]-3-cephem-4-carboxylate

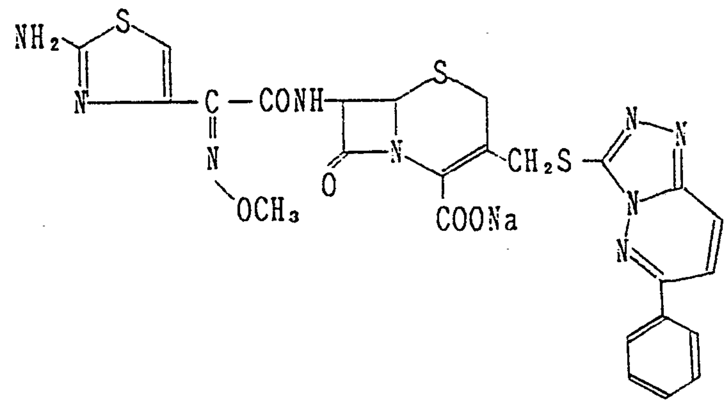

From 7β-[2-(2-aminothiazol-4-yl)-2(Z)-methoxyiminoacetamido]-3-(3-oxobutyryloxy )methyl-3-cephem-4-carboxylic acid and 3-mercapto-6-phenyl-s-triazolo[4,3-b]pyridazine, the title compound was obtained by the same method as described in Example 1.
Elemental analysis for $C_{25}H_{20}N_9O_5S_3Na{\bullet}2H_2O$:
Calcd.(%) : C,44.06; H,3.55; N,18.50
Found (%) : C,44.25; H,3.69; N,18.37
IR $\nu_{max}^{KBr}$ $cm^{-1}$: 1760, 1660, 1600, 1520
NMR($d_6$-DMSO)δ: 3.80(3H,s), 4.40(2H,s), 4.95(1H,d,J=5Hz). 5.60(1H,d.d,J=9Hz & 5Hz), 6.66(1H,s), 7.10-(2H,br.,s), 7.43-7.63(3H,m)

Example 6

Sodium 7β-[2-(2-aminothiazol-4-yl)-2(Z)-methoxyiminoacetamido]-3-[(6-methylthio-s-triazolo[4,3-b]-pyridazin-3-yl)thiomethyl]-3-cephem-4-carboxylate

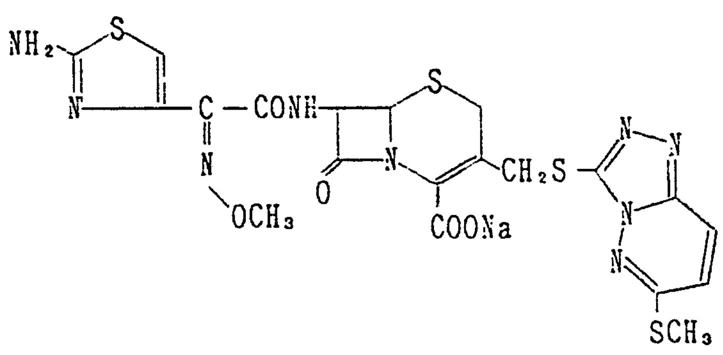

7β-[2-(2-Aminothiazol-4-yl)-2(Z)-methoxyiminoacetamido]-3-(3-oxobutyryloxy)methyl-3-cephem-4-carboxylic acid was reacted with 3-mercapto-6-methylthio-s-triazolo[4,3-b]pyridazine by the same method as described in Example 1 to give the title compound.
Elemental analysis for $C_{20}H_{18}N_9O_5S_4Na{\bullet}3H_2O$:
Calcd. (%) : C,37.22; H,3.75; N,19.53
Found (%) : C,37.40; H,3.61; N,19.29

IR $^{KBr}_{max}$ $cm^{-1}$: 1760, 1670, 1600, 1520, 1030
NMR($d_6$-DMSO)δ: 2.60(3H,s), 3.40 & 3.70(2H,ABq,J = 18Hz), 3.83(3H,s), 4.40(2H,s), 5.00(1H,d,J = 5Hz), 5.60-(1H,d,d,J = 9Hz & 5Hz), 6.70(1H,s), 7.20(2H,br.,s), 7.23(1H,d,J = 9Hz), 8.10(1H,d,J = 9Hz), 9.53(1H,d,J = 9Hz)

Example 7

Sodium 7β-[2-(2-aminothiazol-4-yl)-2(Z)-methoxyiminoacetamido]-3-[(6-methoxy-s-triazolo[4,3-b]-pyridazin-3-yl)thiomethyl]-3-cephem-4-carboxylate

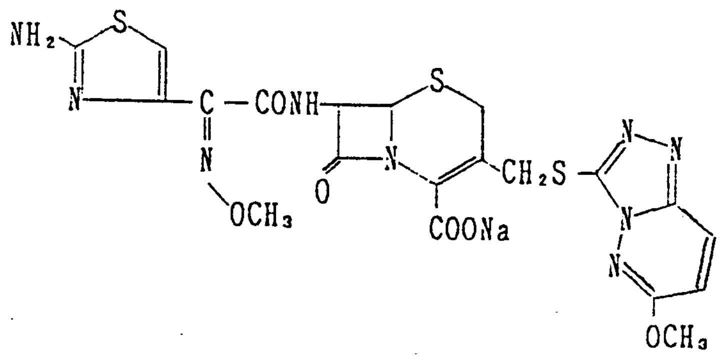

7β-[2-(2-Aminothiazol-4-yl)-2(Z)-methoxyiminoacetamido]-3-(3-oxobutyryloxy)methyl-3-cephem-4-carboxylic acid and 3-mercapto-6-methoxy-s-triazolo[4,3,-b]pyridazine were reacted by the same method as described in Example 1 to give the title compound.
Elemental analysis for $C_{20}H_{18}N_9O_6S_3Na \bullet 2H_2O$:
Calcd. (%) : C,38.90; H,3.59; N,20.42
Found (%) : C,38.69; H,3.35; N,20.73
IR $\nu^{KBr}_{max}$ $cm^{-1}$: 1750, 1650, 1600, 1510
NMR($d_6$-DMSO)δ: 3.65 & 3.73(2H,ABq,J = 18Hz), 3.90(3H,s), 4.06(3H,s), 4.40(2H,s), 5.03(1H,d,J = 5Hz), 5.66-(1H,d.d, J = 9Hz & 5Hz), 6.73(1H,s), 7.06(1H,d,J = 9Hz), 7.20(2H, br.,s), 8.20(1H,J = 9Hz), 9.53(1H,d,J = 9Hz)

Example 8

Sodium 7β-[2-(2-aminothiazol-4-yl)-2(Z)-methoxyiminoacetamido]-3-[(6-fluoro-s-triazolo[4,3-b]pyridazin-3-yl)thiomethyl]-3-cephem-4-carboxylate

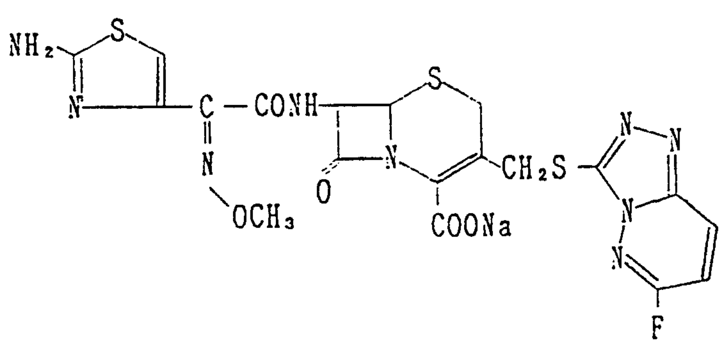

7β-[2-(2-Aminothiazol-4-yl)-2(Z)-methoxyiminoacetamido]-3-(3-oxobutyryloxy)methyl-3-cephem-4-carboxylic acid and 3-mercapto-6-fluoro-s-triazolo[4,3-b]pyridazine were reacted by the same method as described in Example 1 to give the title compound.
Elemental analysis for $C_{19}H_{15}N_9O_5FS_3Na \bullet 2H_2O$:
Calcd.(%) : C,36.60; H,3.07; N,20.22
Found (%) : C,36.67; H,3.22; N,19.81

IR $\nu_{max}^{KBr}$ $cm^{-1}$: 1765, 1680, 1600

NMR($d_6$-DMSO)δ: 3.46 & 3.73(2H,ABq,J=18Hz), 3.90(3H,s), 4.36(2H,s), 5.06(1H,d,J=5Hz), 5.66-(1H,d.d,J=9Hz & 5Hz), 6.73(1H,s), 7.25(2H,s), 7.46(1H,d,J=9Hz), 8.45(1H,d, J=9Hz), 9.60(1H,d,J=9Hz)

Example 9

Sodium 7β-[2-(2-aminothiazol-4-yl)-2(Z)-methoxyiminoacetamido-3-[(6-chloro-s-triazolo[4,3-b]pyridazin-3-yl)thiomethyl]-3-cephem-4-carboxylate

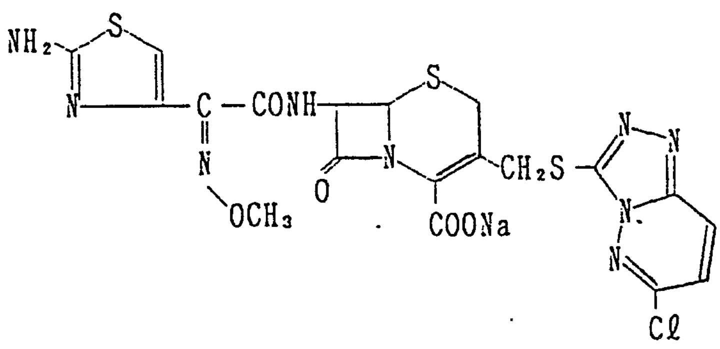

7β-[2-(2-Aminothiazol-4-yl)-2(Z)-methoxyiminoacetamido]-3-(3-oxobutyryloxy)methyl-3-cephem-4-carboxylic acid and 3-mercapto-6-chloro-s-triazolo[4,3-b]pyridazine were reacted by the same method as described in Example 1 to give the title compound.

Elemental analysis for $C_{19}H_{15}N_9O_5ClS_3Na \bullet H_2O$:

Calcd.(%) : C,36.69; H,2.75; N,20,27

Found (%) : C,36.48; H,3.08; N,19.87

IR $\nu_{max}^{KBr}$ $cm^{-1}$: 1760, 1670, 1600

NMR($d_6$DMSO)δ: 3.85(3H,s), 4.40(2H,s), 5.03(1H,d,J=5Hz), 5.63(1H,d,d,J=9Hz & 5Hz), 7.73(1H,s), 7.23-(2H,br.,s), 7.46(1H,d,J=9Hz), 8.40(1H,d,J=9Hz), 9.60(1H,d,J=9Hz)

Example 10

Sodium 7β-[2-(2-aminothiazol-4-yl)-2(Z)-methoxyiminoacetamido]-3-[(s-triazolo[1,5-b]pyridazin-6-yl)-thiomethyl] 3-cephem-4-carboxylate

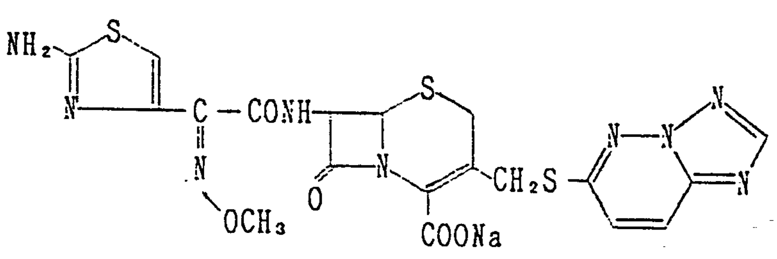

7β-[2-(2-Aminothiazol-4-yl)-2(Z)-methoxyiminoacetamido]-3-(3-oxobutyryloxy)methyl-3-cephem-4-carboxylic acid and 6-mercapto-s-triazolo[1,5-b]pyridazine were reacted by the same method as described in Example 1 to give the title compound.

Elemental analysis for $C_{19}H_{16}N_9O_5S_3Na \bullet 2H_2O$:

Calcd.(%) : C,37.68; H,3.33; N,20.82

Found (%) : C,37.77; H,3.62; N,20.60

IR $\nu_{max}^{KBr}$ $cm^{-1}$: 1760, 1670, 1600, 1530

NMR($d_6$-DMSO)δ: 3.46 & 4.66(2H,ABq,J=18Hz), 3.74(3H,s), 4.35 & 4.57(2H,ABq,J=14Hz), 5.04-(1H,d,J=5Hz), 5.62(1H,d.d,J=9Hz & 5Hz), 6.74(1H,s), 7.20(2H,br.,s), 7.60 (1H,d,J=10Hz), 8.21-(1H,d,J=10Hz), 8.52(1H,s), 9.50(1H,d,J=9Hz)

Example 11

Sodium 7β-[2-(2-aminothiazol-4-yl)-2(Z)-methoxyiminoacetamido]-3-[(s-triazolo[4,3-b]pyridazin-6-yl)-thiomethyl]-3-cephem-4-carboxylate

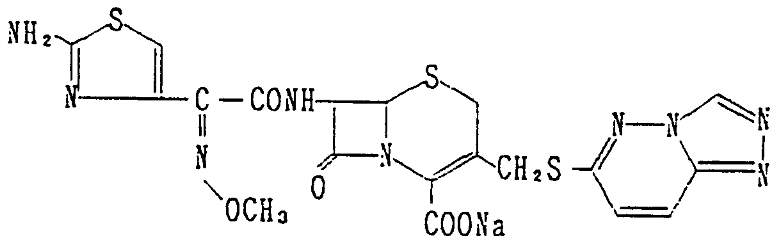

7β-[2-(2-Aminothiazol-4-yl)-2(Z)-methoxyiminoacetamido]-3-(3-oxobutyryloxy)methyl-3-cephem-4-carboxylic acid and 6-mercapto-s-triazolo[4,3-b]pyridazine were reacted by the same method as described in Example 1 to give the title compound.
Elemental analysis for $C_{19}H_{16}N_9O_5S_3Na\bullet2H_2O$:
Calcd. (%) : C,37.68; H,3.33; N,20.82
Found (%) : C,37.76; H,3.55; N,20.42
IR $\nu^{KBr}_{max}$ cm$^{-1}$: 1760, 1670, 1600, 1530
NMR($d_6$-DMSO)δ: 3.34 & 3.66(2H,ABq,J=18Hz), 3.84(3H, s), 4.24 & 4.70(2H,ABq,J=12Hz), 5.02-(1H,d,J=5Hz), 5.60(1H,d•d,J=9Hz & 5Hz), 6.74(1H,s), 7.17(2H,br.,s), 7.22(1H,d,J=10Hz), 8.14-(1H,d,J=10Hz), 9.45(1H,d,J=9Hz), 9.50(1H,s)

Example 12

Sodium 7β-[2-(2-aminothiazol-4-yl)-2(Z)-methoxyiminoacetamido]-3-[(s-triazolo[1,5-a]pyridin-5-yl)-thiomethyl]-3-cephem-4-carboxylate

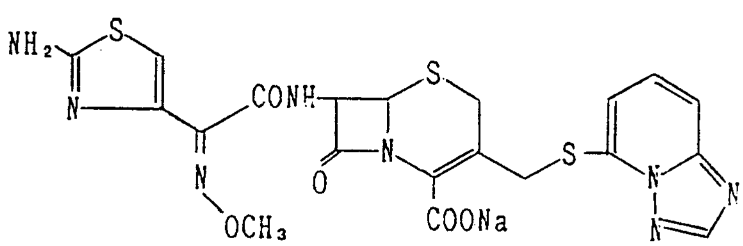

In the same method as described in Example 1, the title compound was obtained from 7β-[2-(2-aminothiazol-4-yl)-2(Z)-methoxyiminoacetamido]-3-(3-oxobutyryloxy)methyl-3-cephem-4-carboxylic acid and 5-mercapto-s-triazolo[1,5-a]pyridine.
Elemental analysis for $C_{20}H_{16}N_8O_5S_3Na\bullet4H_2O$:
Calcd. (%) : C,37.50; H,3.93; N,17.49
Found (%) : C,37.95; H,3.29; N,17.29
IR $\nu^{KBr}_{max}$ cm$^{-1}$ : 1765, 1660, 1610

NMR ($d_6$-DMSO)δ : 3.42 & 3.60(2H,ABq, J=18Hz), 3.82(3H,s), 4.40 & 4.58 (2H,ABq, J=14Hz), 5.0-(1H,d,J=4.5Hz), 5.56(1H,d.d,J=4.5Hz & J=8Hz), 6.72(1H,s), 7.13(2H,br.,), 7.45-7.68(3H,m), 8.47(1H,s), 9.43(1H,d,J=8Hz)

Example 13

Sodium 7β-[2-(2-aminothiazol-4-yl)-2(Z)-methoxyiminoacetamido]-3-[(s-triazolo[4,3-a]pyridin-5-yl)-thiomethyl]-3-cephem-4-carboxylate

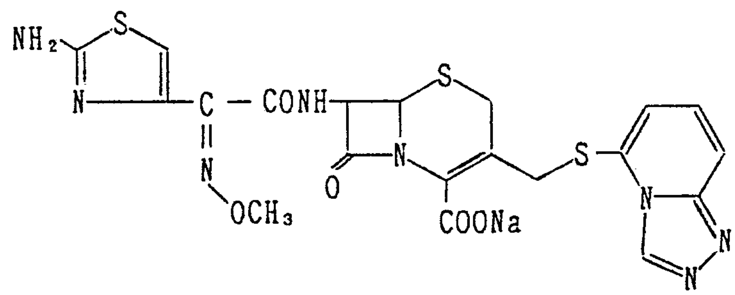

By the same method as described in Example 1, the title compound was obtained from 7β-[2-(2-aminothiazol-4-yl)-2(Z)-methoxyiminoacetamido]-3-(3-oxobutyryloxy)methyl-3-cephem-4-carboxylic acid and 5-mercapto-s-triazolo[4,3-a]pyridine.
Elemental analysis for $C_{20}H_{17}N_8O_5S_3Na \bullet 3H_2O$:
Calcd. (%) : C,38.58; H,3.72; N,18.00
Found (%) : C,58.41; H,3.61; N,18.26
IR $\nu_{max}^{KBr}$ $cm^{-1}$ : 1760, 1670, 1610
NMR ($D_6$-DMSO)δ : 3.85(3H,s), 4.43(2H,s), 5.00(1H,d,J=5Hz), 5.50-5.70(1H,m), 6.73(1H,s), 7.10-7.10-7.30-(3H,m), 7.40(1H,d,J=9Hz), 7.70(1H,d,J=9Hz), 9.33(1H,s), 9.50(1H,d,J=9Hz)

Example 14

Pivaloyloxymethyl 7β-[2-(2-aminothiazol-4-yl)-2(Z)-methoxyiminoacetamido]-3-[(s-triazolo[4,3-b]-pyridazin-6-yl)thiomethyl]-3-cephem-4-carboxylate

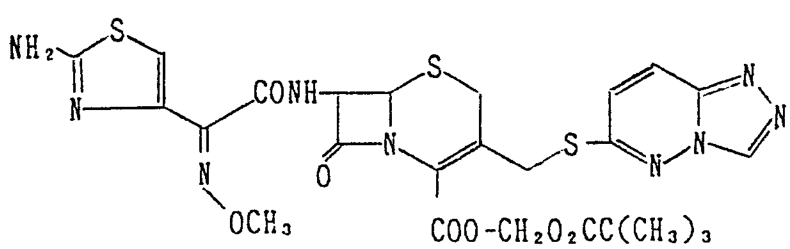

0.3g of sodium 7β-[2-(2-aminothiazol-4-yl)-2(Z)-methoxyiminoacetamido]-3-[(s-triazolo[4,3-b]pyridazin-6-yl)thiomethyl]-3-cephem-4-carboxylate was dissolved in 5 ml of N,N-dimethylformamide and the mixture was cooled to -5°C. 0.6 g of iodomethyl pivalate was added to the stirred mixture which was then stirred for 10 minutes. The reaction mixture was diluted with ethyl acetate (100 ml), washed with cold water, then with saturated sodium chloride solution, and dried over anhydrous magnesium sulfate. The solvent was evaporated under reduced pressure, and to the residue was added isopropyl ether to make a powder. 0.27 g of the title compound was obtained by filtration.
Elemental analysis for $C_{25}H_{26}N_9O_7S_3 \bullet 2H_2O$:
Calcd. (%) : C,43.10; H,4.33; N,18.09
Found (%) : C,43.57; H,4.09; N,17.68

IR $\nu_{max}^{KBr}$ $cm^{-1}$ : 1785, 1580, 1530
NMR ($d_6$-DMSO)$\delta$ : 1.12(9H,s), 3.25(2H,br.,s), 3.82(3H,s), 4.06 & 4.42(2H,ABq,J = 14Hz), 5.16-(1H,d,J = 4.5Hz), 5.78(1H,d.d,J = 4.5Hz & J = 9Hz), 5.87 & 6.03(2H,ABq,J = 6Hz), 6.72(1H,s), 7.12(2H,br.,s), 7.24(1H,d,J = 10Hz), 8.19(1H,d,J = 10Hz), 9.33(1H,s), 9.51(1H,d,J = 9Hz)

Example 15

1-Ethoxycarbonyloxyethyl 7$\beta$-[2-(2-aminothiazol-4-yl)-2(Z)-methoxyiminoacetamido]-3-[(s-triazolo[4,3-b]-pyridazin-6-yl)thiomethyl]3-cephem-4-carboxylate

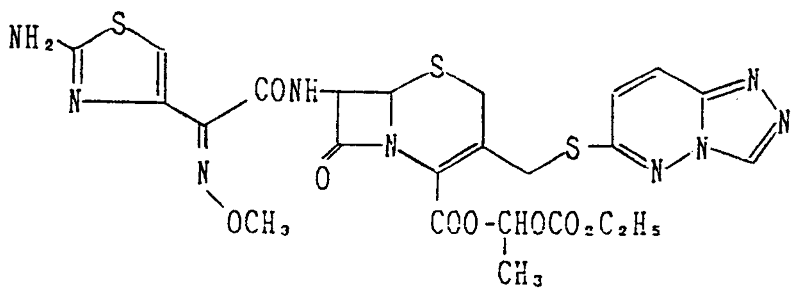

By the same method as described in Example 14, from sodium 7$\beta$-[2-(2-aminothiazol-4-yl)-2(Z)-methoxyiminoacetamido-3-[(s-triazolo[4,3-b]pyridazin-6-yl)thiomethyl]-3-cephem-4-carboxylate and 1-(ethoxycarbonyloxy)ethyl iodide, the title compound was obtained.
Elemental analysis for $C_{24}H_{24}N_9O_8S_3$:
Calcd. (%) : C,43.50; H,3.65; N,19.02
Found (%) : C,43.84; H,4.17; N,18.71
IR $\nu_{max}^{KBr}$ $cm^{-1}$ : 1785, 1655, 1575, 1530
NMR ($d_6$-DMSO)$\delta$ : 1.15 & 1.19(3H,two t,J = 7Hz), 1.53(3H,d,J = 6Hz), 3.27(2H,br.,s), 3.82(3H,s), 3.9-4.3-(2H,m), 4.35 & 4.53(2H,ABq,J = 13Hz), 5.16(1H,d,J = 4.5Hz), 5.79(1H,d.d,J = 4.5Hz & J = 8Hz), 6.71(1H,s), 7.13(1H,d,J = 10Hz), 8.19(1H,d,J = 10Hz), 9.34 & 9.37(1H,two s), 9.50(1H,d,J = 8Hz)

By the similar method as described in Examples 1 to 15, the following compounds (I) can be prepared.

(1) 7$\beta$-[2-(5-amino-1,2,4-thiadiazol-3-yl)-2(Z)-methoxyiminoacetamido]-3-[(s-triazolo[4,3-b]pyridazin-3-yl)thiomethyl]-3-cephem-4-carboxylic acid, or a salt or ester thereof.

(2) 7$\beta$-[2-(5-amino-1,2,4-thiadiazol-3-yl)-2(Z)-carboxymethoxyiminoacetamido]-3-[(s-triazolo[4,3-a]-pyridin-3-yl)thiomethyl]-3-cephem-4-carboxylic acid, or a salt or ester thereof.

(3) 7$\beta$-[2-(5-amino-1,2,4-thiadiazol-3-yl)-2(Z)-methoxyiminoacetamido]-3-[(s-triazolo[1,5-b]pyridazin-6-yl)thiomethyl]-3-cephem-4-carboxylic acid, or a salt or ester thereof.

(4) 7$\beta$-[2-(5-amino-1,2,4-thiadiazol-3-yl)-2(Z)-methoxyiminoacetamido]-3-[(s-triazolo[4,3-b]pyridazin-6-yl)thiomethyl]-3-cephem-4-carboxylic acid, or a salt or ester thereof.

(5) 7$\beta$-[2-(5-amino-1,2,4-thiadiazol-3-yl)-2(Z)-methoxyiminoacetamido]-3-[(s-triazolo[4,3-a]pyridin-5-yl)-thiomethyl]-3-cephem-4-carboxylic acid, or a salt or ester thereof.

## Claims

1. A cephem compound of the general formula:

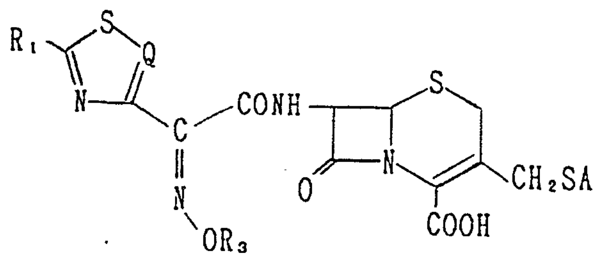

, wherein $R_1$ is an amino group which may be protected,
Q is nitrogen or a group of C-$R_2$ in which $R_2$ is hydrogen or a halogen,
$R_3$ is hydrogen or a hydrocarbon residue which may be substituted,
A is a triazolopyridinyl, triazolopyridazinyl or triazolopyrazinyl group which may be substituted,
, or a salt or ester thereof.

2. The compound of claim 1 in which a triazolopyridine ring constituting the triazolopyridinyl group which may be substituted is one shown as

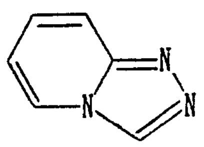 , 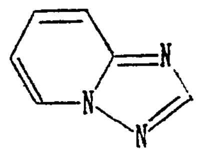 or 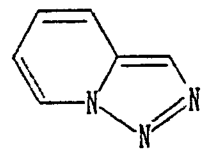

3. The compound of claim 1 in which a triazolopyridazine ring constituting the triazolopyridazine group which may be substituted is one shown as

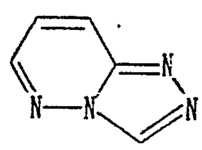 , 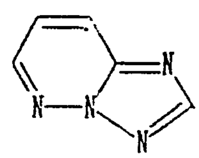 or 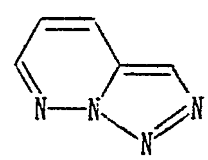

4. The compound of claim 1 in which a triazolopyrazine ring constituting the triazolopyrazinyl group which may be substituted is one shown as

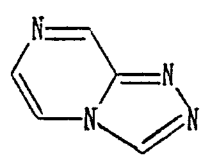 , 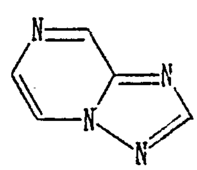 or 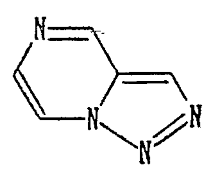

5. The compound of claim 1 in which a substituent on the triazolopyridinyl, triazolopyridazinyl or triazolopyrazinyl group is a phenyl, $C_{1-6}$ alkoxy, $C_{1-6}$ alkylthio, morpholino or halogen.

6. The compound of claim 5 in which the $C_{1-6}$ alkoxy group is methoxy, ethoxy or propoxy.

7. The compound of claim 5 in which the $C_{1-6}$ alkylthio group is methylthio, ethylthio or propylthio.

8. The compound of claim 5 in which the halogen is fluorine, chlorine or bromine.

9. The compound of claim 1 in which A is

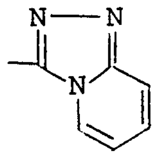 , 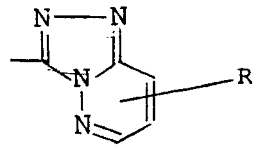

(wherein R is morpholino, phenyl, $C_{1-6}$ alkoxy, $C_{1-6}$ alkylthio or halogen),

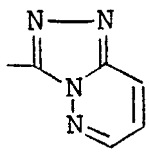 , 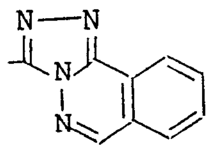 , 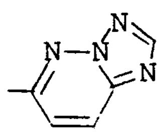 , 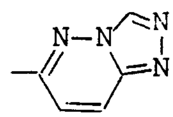 ,

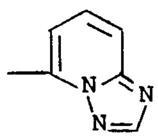 or 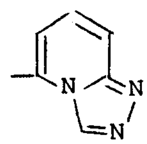 .

10. The compound of claim 1 in which A is

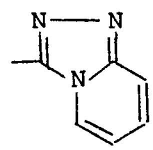 , 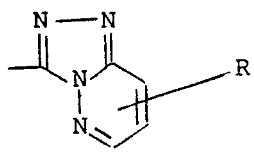

(wherein R is morpholino, phenyl, $C_{1-6}$ alkoxy, $C_{1-6}$ alkylthio or halogen),

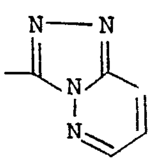 , 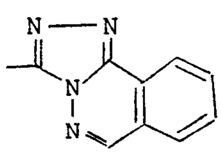 , 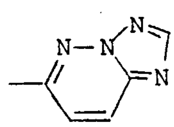 or 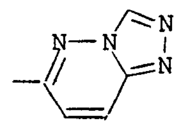 .

11. The compound of claim 1 in which $R_1$ is amino.
12. The compound of claim 1 in which Q is -CH-.
13. The compound of claim 1 in which $R_3$ is $C_{1-3}$ alkyl.
14. The compound of claim 13 in which the $C_{1-3}$ alkyl is methyl, ethyl or propyl.
15. The compound of claim 1 which is:

7β-[2-(2-aminothiazol-4-yl)-2(Z)-methoxyiminoacetamido]-3-[(s-triazolo[4,3-b]pyridazin-3-yl)thiomethyl]-3-cephem-4-carboxylic acid,
7β-[2-(2-aminothiazol-4-yl)-2(Z)-methoxyiminoacetamido]-3-[(s-triazolo[4,3-a]pyridin-3-yl)thiomethyl]-3-cephem-4-carboxylic acid,
7β-[2-(2-aminothiazol-4-yl)-2(Z)-methoxyiminoacetamido]-3-[(s-triazolo[4,3-a]phthalazin-3-yl)thiomethyl]-3-cephem-4-carboxylic acid,
7β-[2-(2-aminothiazol-4-yl)-2(Z)-methoxyiminoacetamido]-3-[(6-morpholino-s-triazolo[4,3-b]pyridazin-3-yl)-thiomethyl]-3-cephem-4-carboxylic acid,
7β[2-(2-aminothiazol-4-yl)-2(Z)-methoxyiminoacetamido]-3-[(6-phenyl-s-triazolo[4,3-b]pyridazin-3-yl)-thiomethyl]-3-cephem-4-carboxylic acid,
7β-[2-(2-aminothiazol-4-yl)-2(Z)-methoxyiminoacetamido]-3-[(6-methylthio-s-triazolo[4,3-b]pyridazin-3-yl)-thiomethyl]-3-cephem-4-carboxylic acid,
7β-[2-(2-aminothiazol-4-yl)-2(Z)-methoxyiminoacetamido]-3-[(6-methoxy-s-triazolo[4,3-b]pyridazin-3-yl)-

thiomethyl]-3-cephem-4-carboxylic acid,
7β-[2-(2-aminothiazol-4-yl)-2(Z)-methoxyiminoacetamido]-3-[(6-fluoro-s-triazolo[4,3-b]pyridazin-3-yl)-thiomethyl]-3-cephem-4-carboxylic acid,
7β-[2-(2-aminothiazol-4-yl)-2(Z)-methoxyiminoacetamido]-3-[(6-chloro-s-triazolo[4,3-b]pyridazin-3-yl)-thiomethyl]-3-cephem-4-carboxylic acid,
7β-[2-(2-aminothiazol-4-yl)-2(Z)-methoxyiminoacetamido]-3-[(s-triazolo[1,5-b]pyridazin-6-yl)thiomethyl]-3-cephem-4-carboxylic acid,
7β-[2-(2-aminothiazol-4-yl)-2(Z)-methoxyiminoacetamido]-3-[(s-triazolo[4,3-b]pyridazin-6-yl)thiomethyl]-3-cephem-4-carboxylic acid,
7β-[2-(2-aminothiazol-4-yl)-2(Z)-methoxyiminoacetamido]-3-[(s-triazolo[1,5-a]pyridin-5-yl)thiomethyl]-3-cephem-4-carboxylic acid,
7β-[2-(2-aminothiazol-4-yl)-2(Z)-methoxyiminoacetamido]-3-[(s-triazolo[4,3-a]pyridin-5-yl)thiomethyl]-3-cephem-4-carboxylic acid or
7β-[2-(2-aminothiazol-4-yl)-2(Z)-methoxyiminoacetamido]-3-[(s-triazolo[4,3-b]pyridin-6-yl)thiomethyl]-3-cephem-4-carboxylic acid,
or a salt or ester thereof.

16. The compound of claim 1 in which the salt is a pharmaceutically acceptable salt.

17. The compound of claim 16 in which the salt is an alkali metal salt.

18. The compound of claim 17 in which the salt is sodium salt.

19. The compound of claim 1 in which the ester is a metabolically unstable, non-toxic ester.

20. The compound of claim 19 in which the ester is an $C_{1-5}$alkanoyloxymethyl ester or an $C_{1-6}$ alkoxycarbonyloxy -$C_{1-6}$alkylester.

21. The compound of claim 20 in which the ester is pivaloyloxymethyl ester.

22. The compound of claim 20 in which the ester is ethoxycarbonyloxyethyl ester.

23. A process for preparing a cephem compound of the general formula:

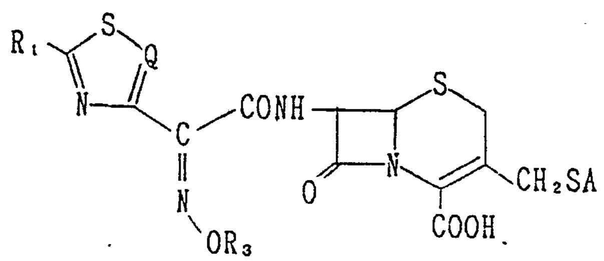

, wherein $R_1$ is an amino group which may be protected,
Q is nitrogen or a group of C-$R_2$ in which $R_2$ is hydrogen or a halogen,
$R_3$ is hydrogen or a hydrocarbon residue which may be substituted,
A is a triazolopyridinyl, triazolopyridazinyl or triazolo pyrazinyl group which may be substituted,
, or a salt or ester thereof,
which comprises

i) reacting a cephem compound of the general formula:

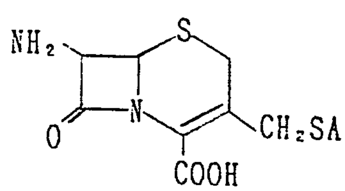

, wherein A is a triazolopyridinyl, triazolopyridazinyl or triazolopyrazinyl group which may be substituted,
, or a salt or ester thereof, with a carboxylic acid of the formula:

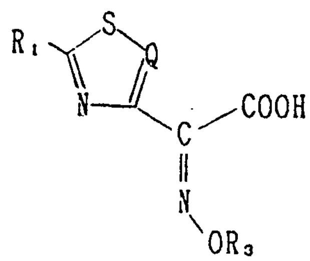

, wherein $R_1$ is an amino group which may be substituted, Q is nitrogen or a group of C-$R_2$ in which $R_2$ is hydrogen or a halogen, $R_3$ is hydrogen or a hydrocarbon residue which may be substituted, or a salt or reactive derivative thereof;

ii) reacting a cephem compound of the general formula:

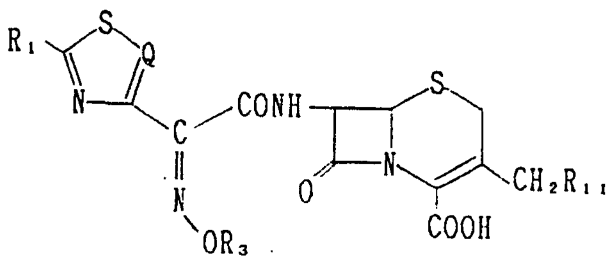

, wherein $R_{11}$ is hydroxy, an acyloxy group, carbamoyloxy, a substituted carbamoyloxy group or a halogen and other symbols are the same as defiend above, salt or ester thereof, with a thiol compound of the general formula ASH in which A is the same as defined above, or a salt thereof;

iii) reacting a cephem compound of the general formula:

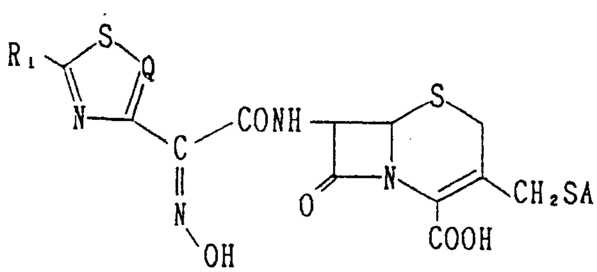

, wherein the symbols are the same as defined above, or a salt or ester thereof, with a compound of the general formula $R_3$'OH in which $R'_3$ is a hydrocarbon residue which may be substituted, or reactive derivative thereof; or

iv) reacting a cephem compound of the general formula:

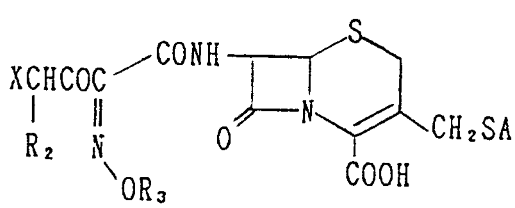

, wherein X is a halogen or a group of $R_6SO_2O$ in which $R_6$ is a lower alkyl group or a phenyl group which may be substituted, and other symbols are the same as defined above, or salt or ester thereof, with a

compound of the general formula $R_1C(=S)NH_2$ in which $R_1$ is the same as defined above; and if needed, removing a protective group of the resultant.

24. The process of claim 23 in which the cephem compound of the general formula is a compound described in any of the claims 2 -22.

25. A pharmaceutical composition which comprises an effective dose of at least one of a cephem compound of the general formula:

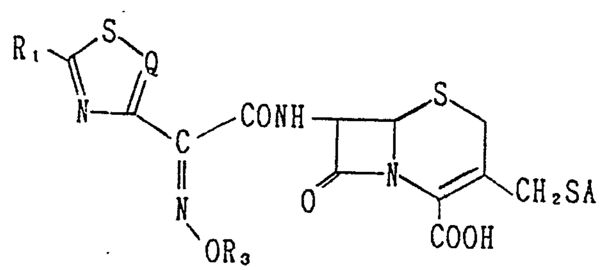

, wherein $R_1$ is an amino group which may be protected,
Q is nitrogen or a group of C-$R_2$ in which $R_2$ is hydrogen or a halogen,
$R_3$ is hydrogen or a hydrocarbon residue which may be substituted,
A is a triazolopyridinyl, triazolopyridazinyl or triazolopyrazinyl group which may be substituted,
or a pharmaceutically acceptable salt or ester thereof, together with a suitable carrier or carriers.

25. The composition of claim 19 in which the cephem compound of the general formula is a compound described in any of the claims 2 -22.